# EUROPEAN PATENT APPLICATION

(11) **EP 2 573 088 A1**
(43) Date of publication of application: **27.03.2013**
(21) Application number: 11382304.1
(22) Date of filing: 26.09.2011
(51) Int. Cl.: C07D 493/04, A61K 31/37, A61K 31/381, A61P 3/10, A61P 3/04, A61P 7/02

(54) **Pyrano[3,2-c]benzopyran-6(2h)-one derivatives and uses thereof**

(71) Applicant: Prous Institute for Biomedical Research, S.A., 08008 Barcelona (ES)
(72) Inventor: Prous, Josep R., E-08008 Barcelona (ES); Serradell, Neus, E-08008 Barcelona (ES); Muñoz, Rosa, E-08008 Barcelona (ES); Flores, Ramón, E-08008 Barcelona (ES); Garçia-Delgado, Noemí, E-08008 Barcelona (ES)
(74) Representative: ABG Patentes, S.L.

(57) **Abstract**

The present invention relates to new pyrano[3,2-c]benzopyran-6(2*H*)-one derivatives that act as SGLT2 inhibitors, which makes them promising candidates for the treatment of diabetes and other diseases and conditions mediated by SGLT2. These new drugs also exhibit platelet aggregation inhibitory activity which makes them promising candidates in the treatment and/or prevention of prothrombotic conditions. The invention also relates to the use of such pyrano[3,2-c]benzopyran-6(2*H*)-one derivatives in the treatment or prevention of the diabetes and other disorders and conditions mediated by SGLT2, as a single active ingredient or in combination with another antidiabetic agent or a drug for the treatment of hypertension, chronic heart failure or atherosclerosis, and pharmaceutical compositions comprising said new pyrano[3,2-*c*]benzopyran-6(2*H*)-one derivatives.

## Description

### FIELD OF THE INVENTION

The present invention relates to pyrano[3,2-c]benzopyran-6(2H)-one derivatives and their use in the preparation of pharmaceutical compositions for treating diabetes and other diseases and conditions mediated by SGLT2. These new drugs also exhibit platelet aggregation inhibitory activity which makes them promising candidates in the treatment and/or prevention of prothrombotic conditions.

### BACKGROUND OF THE INVENTION

The World Health Organization (WHO) estimates that approximately 200 million people worldwide have diabetes, and the incidence is expected to increase to about 360 million by 2030, particularly in the developing countries. Diabetes is a metabolic disorder characterized by hyperglycemia, which can lead to serious complications, i.e., cardiovascular disease, renal failure, retinal, nerve and microvascular damage, as well as obesity. Approximately 90% of patients have type 2 diabetes, a chronic disorder characterized by increased hepatic glucose output, beta cell dysfunction, impaired insulin secretion and insulin resistance. Diet and exercise are the initial strategies for managing type 2 diabetes, but a significant proportion of patients do not respond adequately in the long term and require insulin therapy or other treatments, including thiazolidinediones, sulfonylureas, biguanides, α-glucosidase inhibitors, meglitinides and dipeptidyl peptidase 4 (DPP IV) inhibitors. Metformin, a biguanide, is the usual initial therapy for type 2 diabetes but is associated with gastrointestinal side effects [see Jabbour, S.A., Goldstein, B.J. Int J Clin Pract 2008, 62(8): 1279-84; Isaji, M. Curr Opin Investig Drugs 2007, 8(4): 285-92].

There is a need for novel, more effective and safer therapies for type 2 diabetes. The sodium/glucose cotransporter 2 (SGLT2) is a promising target for therapeutic intervention in type 2 diabetes. SGLT2 inhibitors have been described for the treatment of type 2 diabetes, metabolic syndrome and obesity [Idris, I., Donnelly, R. Diabetes Obes Metab 2009, 11(2): 79-88; Isaji, M. Curr Opin Investig Drugs 2007, 8(4): 285-92].

Two different isoforms of the sodium/glucose cotransporter family (*SLC5A*) have been identified: the high-affinity low-capacity sodium/glucose cotransporter 1 (SGLT1) and the low-affinity high-capacity sodium/glucose cotransporter 2 (SGLT2). Whereas SGLT1 is located mainly in the intestine, as well as the heart and kidney, and accounts for only about 10% of glucose reabsorption in the kidney, SGLT2 is present in the S1 and S2 segments of the renal proximal tubule and is responsible for about 90% of total renal glucose reabsorption. SGLT1 cotransports glucose and Na+ in a 1:2 ratio, whereas SGLT2 transports glucose and Na+ in a 1:1 ratio. Compounds that inhibit SGLT2 are therefore expected to normalize plasma glucose by promoting the excretion of glucose in the urine (kidneys) and thereby reducing the accumulation of glucose in the body, while preserving insulin secretion, as well as leading to weight loss. Moreover, such compounds do not induce hypoglycemia and appear to have a good safety profile [see Washburn, W.N. Expert Opin Ther Patents 2009, 19(11): 1485-99; Jabbour, S.A., Goldstein, B.J. Int J Clin Pract 2008, 62(8): 1279-84; Neumiller, J.J. et al. Drugs 2010, 70(4): 377-8; Isaji, M. Curr Opin Investig Drugs 2007, 8(4): 285-92; Asano, T. et al. Drugs Fut 2004, 29(5): 461-6].

Most SGLT2 inhibitors are glycosides (*O*-, *C*-, *S*-, and *N*-glycosides) based on the natural compound phlorizin, a nonselective SGLT inhibitor long known to reduce plasma glucose and improve insulin resistance by increasing renal glucose secretion, but with limited therapeutic potential due to poor oral bioavailability and absorption from the gastrointestinal tract [Goldstein, B.J. Int J Clin Pract 2008, 62(8): 1279-84; Isaji, M. Curr Opin Investig Drugs 2007, 8(4): 285-92; Asano, T. et al. Drugs Fut 2004, 29(5): 461-6]. Several compounds are in late-stage development, including Bristol-Myers Squibb's dapagliflozin, which has been submitted for approval in the E.U. and the U.S., and Johnson & Johnson's canagliflozin, currently in phase III trials.

Phlorizin, a β-D-glucoside isolated from the root bark of the apple tree, was the first nonselective SGLT inhibitor and consists of a glucose moiety and an aglycone in which two aromatic carbocycles are joined by an alkyl spacer. T-1095 is the prodrug of T-1095A, which is a nonselective SGLT inhibitor that is a derivative of phlorizin and categorized as an aromatic hydrocarbon *O*-glycoside. Thereafter, nonphlorizin *O-*glycoside derivatives selectively inhibiting SGLT2 have been described, the best example being sergliflozin, a prodrug of sergliflozin-A, which is an aromatic *O-*glycoside.

Heteroaromatic *O*-glycosides have also been described. A lead compound, remogliflozin etabonate, a prodrug of remogliflozin, is a heteroaromatic *O*-glycoside.

The next step in the development of SGLT2 inhibitors was based on aromatic and heteroaromatic *C*-glycosides, in which the glucose moiety binds to the aglycone directly through a carbon-carbon bond. Dapagliflozin is a representative compound of aromatic *C*-glycosides as it enhances the chemical stability of the glycosidic bond. *C-*Glycosides are more metabolically stable than *O*-glycosides due to their resistance to gastrointestinal β-glucosidases, and are rapidly absorbed in the gastrointestinal tract without modification of the prodrug form. *N*-glycosides, thioglycosides, as well as modified sugar rings developed for combination with various aglycone structures, have also been described.

Another important group of structures claimed as SGLT2 inhibitors are those with a bicyclic sugar ring containing a spirocycle.

Nevertheless, the art fails to disclose bicyclic sugar rings containing a fused ring as SGLT2 inhibitors.

### DESCRIPTION OF THE INVENTION

The inventors have surprisingly obtained new pyrano[3,2-c]benzopyran-6(2*H*)-one derivatives showing SGLT2-inhibitory activity. This activity makes them promising candidates for the treatment of diabetes and other diseases and conditions mediated by SGLT2. Advantageously, these new drugs also possess platelet aggregation-inhibitory activity.

In one aspect, the present invention provides pyrano[3,2-*c*]benzopyran-6(2*H*)-one derivatives of formula (I) 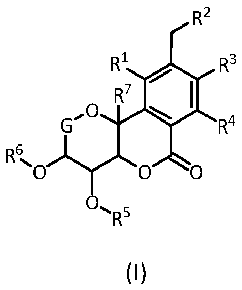 wherein
**R¹** and **R⁴** are independently selected from the group consisting of -H; halogen; -OH; C₁-C₈ alkyl optionally mono- or polysubstituted by halogen; C₁-C₈ alkyloxy optionally mono- or polysubstituted by halogen; C₃-C₁₀ cycloalkyl; -NO₂; -CN; -CO₂H; -C(O)OC₁-C₈ alkyl; -SO₂C₁-C₈ alkyl; and a 5-, 6-, or 7-membered heterocyclyl group which contains from 1 to 2 heteroatoms in the ring independently selected from the group consisting of N, O, and S;
**R²** is selected from the group consisting of C₆-C₁₀ aryl; C₃-C₁₀ cycloalkyl; a 5- or 6-membered monocyclic heteroaryl group which contains from 1 to 4 heteroatoms in the ring independently selected from the group consisting of N, O, and S; an 8-, 9-, or 10-membered bicyclic heteroaryl group which contains from 1 to 4 heteroatoms independently selected from the group consisting of N, O, and S; and a 5-, 6-, or 7-membered heterocyclyl group which contains from 1 to 2 heteroatoms in the ring independently selected from the group consisting of N, O, and S; wherein
R² is optionally substituted by a group selected from halogen; -OH; C₁-C₈ alkyl optionally mono- or polysubstituted by halogen; C₂-C₈ alkenyl; C₂-C₈ alkynyl; C₁-C₈ alkyloxy optionally mono- or polysubstituted by halogen; C₃-C₁₀ cycloalkyl, a C₆-C₁₀ aryl group which is optionally mono- or disubstituted by halogen; -CN; -NO₂; -CO₂H; - C(O)OC₁-C₈ alkyl; and -SO₂C₁-C₈ alkyl; a 5- or 6-membered monocyclic heteroaryl group which contains from 1 to 4 heteroatoms in the ring independently selected from the group consisting of N, O, and S; and an 8-, 9-, or 10-membered bicyclic heteroaryl group which contains from 1 to 4 heteroatoms in the ring independently selected from the group consisting of N, O, and S;
**R³** is selected from the group consisting of -H; halogen; C₁-C₈ alkyl optionally mono- or polysubstituted by halogen; C₂-C₈ alkenyl; C₂-C₈ alkynyl; C₁-C₈ alkyloxy optionally mono- or polysubstituted by halogen; hydroxy-C₁-C₈ alkyl; C₃-C₁₀ cycloalkyl; -OH; -NO₂; -CN; -CO₂H; -C(O)OC₁-C₈ alkyl; -SO₂C₁-C₈ alkyl; and a 5-, 6-, or 7-membered heterocyclyl group which contains from 1 to 2 heteroatoms in the ring independently selected from the group consisting of N, O, and S;
**R⁵** and **R⁶** are independently selected from the group consisting of -H; -C(O)C₁-C₈ alkyl; and -C(O)OC₁-C₈ alkyl;
**R⁷** is selected from the group consisting of-H and -OH;
**G** is selected from the group consisting of 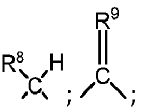 and 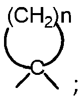 wherein
**R⁸** is selected from the group consisting of -CH₂OH; -CH₂OC₁-C₈ alkyl; -CO₂H; -C(O)OC₁-C₈ alkyl; -CH₂OC(O)-C₁-C₈ alkyl; -CH₂OC(O)O-C₁-C₈ alkyl; and C₁-C₈ alkyl optionally mono-or polysubstituted by halogen;
**R⁹** is selected from the group consisting of C₁-C₈ alkylidene; and C₃-C₁₀ cycloalkylidene;
**n** is an integer selected from 2, 3, and 4;
or a pharmaceutically acceptable salt or stereoisomer thereof.

In one embodiment, the present invention provides a compound of formula (I), wherein R¹ is selected from the group consisting of -H; -F; -Cl; -Br; -OH; -CN; -NO₂; and C₁-C₄alkyloxy optionally mono- or polysubstituted by -F; C₁-C₄alkyl optionally mono- or polysubstituted by -F. Preferably, R¹ is -H.

In another embodiment, the present invention provides a compound of formula (I), wherein R² is selected from the group consisting of C₆-C₁₀ aryl; a 5- or 6-membered monocyclic heteroaryl group which contains from 1 to 2 heteroatoms in the ring independently selected from the group consisting of N, O, and S; and an 8-, 9-, or 10-membered bicyclic heteroaryl group which contains from 1 to 4 heteroatoms independently selected from the group consisting of N, O, and S; and wherein said R² group is optionally substituted by a group selected from C₁-C₄ alkyl; C₂-C₄ alkenyl; C₂-C₄ alkynyl; C₁-C₄ alkyloxy; C₃-C₆ cycloalkyl; and C₆-C₁₀ aryl; wherein said aryl group is optionally mono- or disubstituted by halogen. Preferably, R² is selected from phenyl; azulen-2-yl; thien-2-yl; and benzo[*b*]thien-2-yl; wherein R² is optionally substituted by a group selected from ethyl; ethynyl; ethoxy; methoxy; cyclopropyl; and 4-fluorophenyl. More preferably, R² is phenyl or thien-2-yl; wherein said R² group is optionally substituted by a group selected from ethyl; ethoxy; methoxy; and 4-fluorophenyl. Even more preferably, R² is selected from a phenyl group substituted by ethyl; ethoxy; or methoxy; or a thien-2-yl group substituted by 4-fluorophenyl.

In another embodiment, the present invention provides a compound of formula (I), wherein R³ is selected from the group consisting of -H; halogen; C₁-C₄ alkyl; -CN; C₃-C₆ cycloalkyl; and C₁-C₄ alkyloxy; wherein said alkyl an alkyloxy groups are optionally mono- or polysubstituted by halogen. More preferably R³ is -H, -Cl or C₁-C₄ alkyl, even more preferably, R³ is -H, -Cl or methyl.

In another embodiment, the present invention provides a compound of formula (I), wherein R⁴ is selected from the group consisting of -H; C₁-C₄ alkyl; halogen; C₁-C₄ alkyloxy; and -OH. Preferably, R⁴ is -H; methyl; halogen; methoxy; or -OH. Even more preferably, R⁴ is -H.

In yet another embodiment, the present invention provides a compound of formula (I), wherein R⁵ is -H.

In yet another embodiment, the present invention provides a compound of formula (I), wherein R⁶ is -H.

In yet another embodiment, the present invention provides a compound of formula (I), wherein R⁷ is -H.

In yet another embodiment, the present invention provides a compound of formula (I), wherein G is selected from the group consisting of:
(a) 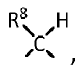 wherein R⁸ is -CH₂OH; -CH₂OC₁-C₄ alkyl; or -CH₂OC(O)OC₁-C₄ alkyl;
(b) 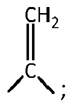 and
(c) 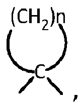 wherein n is an integer selected from 2, 3, or 4.

Preferably, G is selected from the group consisting of:
(a) 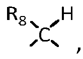 wherein R⁸ is -CH₂OH or -CH₂OC(O)OCH₂CH₃;
(b) 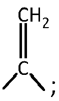 and
(c) 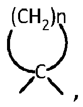 wherein n is 2.

In yet another embodiment, the present invention provides a compound of formula (I), selected from the group consisting of:
- (2*R*,3*S*,4*S*,4a*R*,10b*S*)-9-(4-Ethylbenzyl)-3,4-dihydroxy-2-(hydroxymethyl)-3,4,4a,10b-tetrahydropyrano[3,2-*c*][2]benzopyran-6(2*H*)-one;
- (2*R*,3*S*,4*S*,4a*R*,10b*S*)-8-Chloro-9-(4-ethoxybenzyl)-3,4-dihydroxy-2-(hydroxymethyl)-3,4,4a,10b-tetrahydropyrano[3,2-*c*][2]benzopyran-6(2*H*)-one;
- (2*R*,3*S*,4*S*,4a*R*,10b*S*)-8-Chloro-9-(4-ethylbenzyl)-3,4-dihydroxy-2-(hydroxymethyl)-3,4,4a,10b-tetrahydropyrano[3,2-*c*][2]benzopyran-6(2*H*)-one;
- (2*R*,3*S*,4*S*,4a*R*,10b*S*)-9-(4-Ethoxybenzyl)-3,4-dihydroxy-2-(hydroxymethyl)-3,4,4a,10b-tetrahydropyrano[3,2-*c*][2]benzopyran-6(2*H*)-one;
- (2*R*,3*S*,4*S*,4a*R*,10b*S*)-9-(4-Ethylbenzyl)-3,4-dihydroxy-2-(ethoxycarbonyloxymethyl)-3,4,4a,10b-tetrahydropyrano[3,2-*c*][2]benzopyran-6(2*H*)-one; and
- (2*R*,3*S*,4*S*,4a*R*,10b*S*)-3,4-Dihydroxy-2-(hydroxymethyl)-9-(4-methoxybenzyl)-3,4,4a,10b-tetrahydropyrano[3,2-*c*][2]benzopyran-6(2*H*)-one.
or a pharmaceutically acceptable salt or stereoisomer thereof.

In a second aspect, the present invention provides a compound of Formula (I) as defined above, or a pharmaceutically acceptable salt or stereoisomer thereof, for use as a medicament.

### Pharmacological Activity

Compounds of Formula (I) are inhibitors of SGLT2. They also show platelet aggregation-inhibitory activity (see Biological Assays).

Since antidiabetic drugs are used extensively and for prolonged periods in humans, their potential for carcinogenicity when given alone or in combination with other agents is an important criterion for the selection of such drugs.

It has recently been suggested that the use of pioglitazone, a peroxisome proliferator-activated receptor PPAR-γ agonist launched in 1999, may be associated with an increased risk of bladder cancer [Stephenson, J. JAMA 2011, 301(2): 143]. Dapagliflozin, an SGLT2 inhibitor, has been reported to increase the risk for breast and bladder cancer in diabetic patients [FDA Briefing Document. NDA 202293. Advisory Committee Meeting, July 19, 2011]. Two newer drugs, sitaglitin (DPP IV inhibitor) and exenatide (glucagon-like petide analog), launched for the treatment of type 2 diabetes could be linked to a significant increased risk of developing pancreatitis and pancreatic cancer [Elashoff, M. Gastroenterology 2011, 141(1): 150-6]. It has also been reported that there may be a link between diabetes and breast cancer [Schott, S. et al. Exp Clin Endocrinol Diabetes 2010, 118(10): 673-7].

It is therefore necessary to maximize precautions, and for this reason we have submitted representative compounds of formula (I) to carcinogenicity studies as described in the experimental examples. No carcinogenicity was observed in this assay.

The invention provides a method for the treatment of diseases or conditions mediated by SGLT2, comprising the administration of a therapeutically effective amount of a compound of formula (I) as defined above, or a pharmaceutically acceptable salt or stereoisomer thereof.

The compounds of the invention are useful as both therapeutic and prophylactic treatments for diseases or conditions mediated by SGLT2, including diabetes, particularly type 2 diabetes, diabetic complications such as retinopathy, neuropathy, and nephropathy, as well as hyperglycemia, insulin resistance, metabolic syndrome, hyperinsulinemia, hypertension, hyperuricemia, edema, dyslipidemia, chronic heart failure, atherosclerosis, and obesity.

Due to the platelet aggregation-inhibitory activity of compounds of formula (I) as defined above, they can also be used in thrombotic states. Type 2 diabetes is characterized by a procoagulant condition. The high incidence of cardiovascular ischemic events in type 2 diabetic patients is explained by the development of atherothrombotic lesions.

Thus, in a third aspect, the present invention provides a compound of formula (I) as defined above, or a pharmaceutically acceptable salt or stereoisomer thereof, for use in the treatment and/or prevention of a disease or condition selected from the group consisting of diabetes, type 2 diabetes, diabetic complications selected from retinopathy, neuropathy, and nephropathy, hyperglycemia, insulin resistance, metabolic syndrome, hyperinsulinemia, hypertension, hyperuricemia, edema, dyslipidemia, chronic heart failure, atherosclerosis, obesity, and thrombosis. Preferably, the disease or condition is type 2 diabetes, obesity, or thrombosis. More preferably, the disease or condition is type 2 diabetes.

A fourth aspect of the present invention provides a method of treatment and/or prophylaxis of a disease or condition selected from the group consisting of diabetes, type 2 diabetes, diabetic complications selected from retinopathy, neuropathy, and nephropathy, hyperglycemia, insulin resistance, metabolic syndrome, hyperinsulinemia, hypertension, hyperuricemia, edema, dyslipidemia, chronic heart failure, atherosclerosis, obesity, and thrombosis, comprising administering to a subject in need thereof a therapeutically effective amount of a compound of formula (I) as defined above, or a pharmaceutically acceptable salt or stereoisomer thereof.

### Pharmaceutical compositions/formulations and administration

In a fifth aspect, the present invention provides a pharmaceutical composition comprising an effective amount of a compound of formula (I) as defined above, or a pharmaceutically acceptable salt or stereoisomer thereof, and one or more pharmaceutically suitable excipients.

Compounds of the invention may be administered by the oral, sublingual, parenteral, subcutaneous, intramuscular, intravenous, transdermal, intranasal, intraocular, and/or rectal routes. The compounds may be administered alone or in combination with one or more other compounds of the invention or one or more other drugs. In general, the compounds should be administered as a formulation in association with one or more pharmaceutically acceptable excipients.

Thus, in a sixth aspect, the present invention provides a combination comprising compounds of Formula (I) as defined above, or a pharmaceutically acceptable salt or stereoisomer thereof, an another antidiabetic agent selected from the group consisting of biguanides, such as metformin and buformin; sulfonylureas, such as glibenclamide, tolbutamide, and glimepiride; meglitinides, such as nateglinide, repaglinide, and mitiglinide; thiazolidinediones, such as rosiglitazone, pioglitazone, and rivoglitazone; α-glucosidase inhibitors, such as acarbose, voglibose, and miglitol; insulin and insulin analogues, such as biphasic insulin aspart and insulin glargine; glucagon-like peptide 1 (GLP-1) and analogues, such as exenatide, liraglutide, taspoglutide, albiglutide, lixisenatide, and dulaglutide; dipeptidyl peptidase 4 (DPP IV) inhibitors, such as alogliptin, linagliptin, saxagliptin, sitagliptin, and vildagliptin; HMG-CoA reductase inhibitors, such as simvastatin, atorvastatin, lovastatin, rosuvastatin, pravastatin, and fluvastatin; GPR40 agonists, such as TAK-875; GPR119 agonists, such as PSN-821, SAR-260093, and GSK-1292263AA; GPR120 agonists; and glucokinase activators, such as piragliatin and RO-0281675.

In a further aspect, the present invention provides a combination comprising a compound of formula (I) as defined above, or a pharmaceutically acceptable salt or stereoisomer thereof, and at least a drug for the treatment of hypertension, chronic heart failure, or atherosclerosis, selected from the group consisting of angiotensin II receptor antagonists, such as losartan, valsartan, olmesartan, eprosartan, irbesartan, candesartan, and telmisartan; angiotensin-converting enzyme (ACE) inhibitors, such as captopril, enalapril, ramipril, lisinopril, benazepril, and perindopril; endothelin-converting enzyme (ECE) inhibitors, such as daglutril; diuretics, such as torasemide, cicletanine, chlorothiazide, chlortalidone, bendroflumethiazide, clopamide, indapamide, xipamide, hydrochlorothiazide, and amiloride; β-adrenoceptor blockers, such as propranolol, timolol, befunolol, mepindolol, nadolol, levobunolol, levobetaxolol, labetalol, sotalol, and penbutolol; calcium antagonists, such as felodipine, nisoldipine, lercanidipine, bepridil, nicardipine, nitrendipine, nimodipine, verapamil, nifedipine, barnidipine, and amlodipine; α₁-adrenoceptor antagonists, such as doxazosin, prazosin, terazosin, bunazosin, and urapidil; neutral endopeptidase inhibitors, such as ecadotril, candoxatril, and omapatrilat; and renin inhibitors, such as aliskiren.

The compounds of formula (I) of the invention can be prepared as shown in the following reaction schemes and the description thereof, wherein temperatures are expressed in degrees Celsius.

In a first approach, compounds of formula (I) can be prepared following the synthetic pathway depicted in Scheme 1.

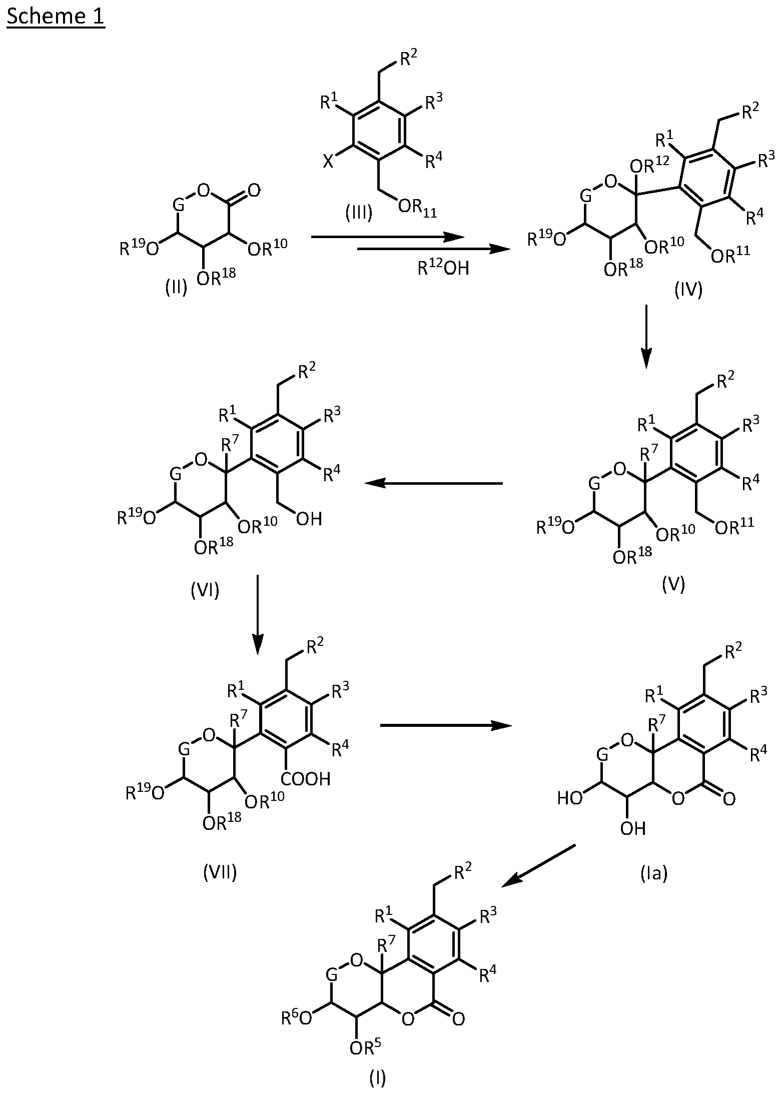

The key intermediate (IV) can be synthesized by means of a nucleophilic addition of (III) (wherein R¹¹ is a hydroxyl protecting group such as allyl, *p*-methoxyphenyl, or *p-*methoxybenzyl and X is selected from Br and I) to the lactone (II) followed by treatment with a compound of formula R¹²OH, wherein R¹² is a hydrogen atom or a methyl group, i.e. treatment with water or methanol in the presence of an acid such as ammonium chloride, methanesulfonic acid, toluenesulfonic acid, sulfuric acid, or hydrochloric acid to yield the intermediate (IV).

When R¹⁰, R¹⁸ and R¹⁹ are hydroxyl protecting groups such as benzyl, methyl, ethyl, methoxymethyl, 2-methoxyethoxymethyl, trialkylsilyl, and the like; the reaction may be carried out in the presence of a lithium donor, for example, *n-, sec-,* or *tert*-butyl lithium. These reactions are preferably conducted between -100 and 0 ºC, particularly preferably between -80 and -10 ºC, in an inert solvent or mixtures thereof, such as tetrahydrofuran, diethyl ether, toluene, heptane, or dichloromethane.

When R¹⁰, R¹⁸ and R¹⁹ are hydroxyl protecting groups such as acyl (preferably acetyl or benzoyl), alkoxycarbonyl, but also benzyl, methyl, ethyl, and the like, the reactions may be carried out in the presence of a Grignard reagent such as isopropylmagnesium bromide or diisopropylmagnesium. These reactions are preferably conducted between -80 and 40 ºC, particularly preferably between 0 and 20 ºC, in an inert solvent or mixtures thereof, such as tetrahydrofuran, diethyl ether, toluene, heptane, or dichloromethane.

The product (V), when R⁷ is -H, is obtained by a reductive removal of the resulting lactol or ketal (IV), coming from the previous reaction, with a reducing agent in the presence of a Lewis or Brönsted acid. Suitable reducing agents include silanes such as triethyl-, triisopropyl-, or triphenylsilane, boranes, lithium aluminum hydride, diisobutylaluminum hydride, sodium borohydride, or sodium cyanoborohydride. The reactions are performed without or in the presence of a Lewis acid such as boron trifluoride etherate, tin tetrachloride, titanium tetrachloride, trimethylsilyltriflate, or zinc iodide, or a Brönsted acid such as hydrochloric acid, acetic acid, or trifluoroacetic acid. The reductions are carried out in a solvent or mixtures thereof, such as dichloromethane, chloroform, acetonitrile, dioxane, tetrahydrofuran, hexane, toluene, ethanol, or water at temperatures between -80 and 30 ºC.

The intermediate (VI) can be prepared by cleavage of the protecting group R¹¹ of (V). Thus, when R¹¹ is allyl, the cleavage is achieved upon treatment with PdCl₂ and sodium acetate in acidic aqueous media at 25 to 80 ºC, with catalytic Pd(PPh₃)₄ and K₂CO₃ in refluxing methanol, or by using any of the standard methods to cleave the allyl group known to those skilled in the art [see Greene, T.W., Wuts, P.G.M. Protective Groups in Organic Synthesis (Third Edition), J. Wiley & Sons, 1999; Vutukuri, D.R. et al. J Org Chem 2003, 68(3): 1146-50]. On the other hand, when R¹¹ is *p*-methoxyphenyl or *p-*methoxybenzyl, the cleavage is achieved upon oxidative conditions with cerium ammonium nitrate in acetonitrile, with dichlorodicyanoquinone in dichloromethane, or by using any of the standard methods to cleave these protecting groups known to those skilled in the art [see Greene, T.W., Wuts, P.G.M. Protective Groups in Organic Synthesis (Third Edition), J. Wiley & Sons, 1999].

The subsequent oxidation of the benzyl alcohol (VI) to the benzoic acid (VII) can be carried out with Jones Reagent (CrO₃ in H₂SO₄), pyridinium clorochromate, potassium permanganate, and the like in a solvent such as acetone, water, tetrahydrofuran, methanol, or dichloromethane. The reactions are preferably conducted between 0 and 20 ºC.

When R¹⁰, R¹⁸ and R¹⁹ are benzyl, the lactonization of the species (VII) to the desired structure (Ia) can be achieved in the presence of a metal catalyst such as PdCl₂, Pd(OH)₂, palladium/carbon, or platinum/carbon in a hydrogen atmosphere (from 1 to 5 bar) in a suitable solvent or mixtures thereof such as methanol, ethyl acetate, and the like. Furthermore, when R¹⁰, R¹⁸ and R¹⁹ are methyl, ethyl, or benzyl the lactonization can be achieved upon treatment with a Lewis acid such as BCl₃, BBr₃, or AlCl₃. The reactions are preferably conducted between -100 and 0 ºC, particularly preferably between -80 and -10 ºC, in an inert solvent or mixtures thereof, such as dichloromethane, tetrahydrofuran, toluene, or hexane. When R¹⁰, R¹⁸ and R¹⁹ are methoxymethyl, 2-methoxyethoxymethyl, or trialkylsilyl the lactonization of the species (VII) can be performed by treatment with methanesulfonic acid, toluenesulfonic acid, sulfuric acid, or hydrochloric acid in a solvent such as methanol, tetrahydrofuran, dichloromethane, and the like. Finally, when R¹⁰, R¹⁸ and R¹⁹ are acyl or alkoxycarbonyl the lactonization of the species (VII) to the desired structure (Ia) can be performed by treatment with sodium methoxide, lithium hydroxide and the like in a solvent such as methanol, tetrahydrofuran and the like.

When R⁵ and/or R⁶ are acyl or alcoxycarbonyl the corresponding species (I) are obtained from compounds (Ia) using standard methodologies known to those skilled in the art.

Alternatively, the synthesis of the key intermediate (V) can be achieved using the methodology depicted in Scheme 2. 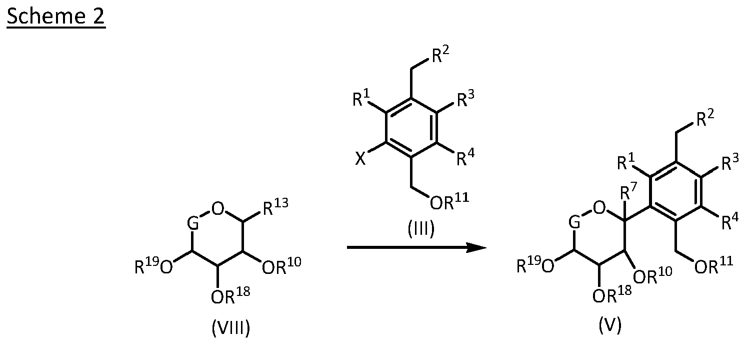 C-glycoside (V) (wherein R⁷ is a hydrogen atom) can be prepared by reaction of the aryl halide (III) (wherein X is halide, ZnCl, ZnBr, or Znl, and R¹¹ is a hydroxyl protecting group such as allyl, *p*-methoxyphenyl, or *p*-methoxybenzyl) and a conveniently substituted compound of formula (VIII), wherein R¹³ may be Cl, Br, I, acetyl, hydroxyl, methoxy, trichloroacetamide, or any other leaving group, and R¹⁰, R¹⁸ and R¹⁹ are hydroxyl protecting groups such as benzyl, methyl, ethyl, methoxymethyl, trialkylsilyl, acyl (preferably acetyl or benzoyl), alkoxycarbonyl, and the like. Thus, when X is Cl, Br, or I the reaction takes place in the presence of Mg in a solvent such as diethyl ether or tetrahydrofuran. On the other hand, when X is ZnCl, ZnBr, or Znl a Negishi cross-coupling reaction can be conducted in the presence of an Ni catalyst such as NiCl₂, NiBr₂, or Ni(COD)₂, and a suitable ligand such as diglyme or 2,6-bis[(4R)-4-phenyl-2-oxazolinyl]pyridine (PyBox), using a solvent such as dimethylformamide, dimethylacetamide, 1,3-dimethyl-2-imidazolidinone, or tetrahydrofuran [see Gong, H., Gagné, M.R. J Am Chem Soc 2008, 130(36): 12177-83].

A second strategy to prepare the compounds of formula (I) can be followed by using an aromatic ketal (IX), as shown in Scheme 3.

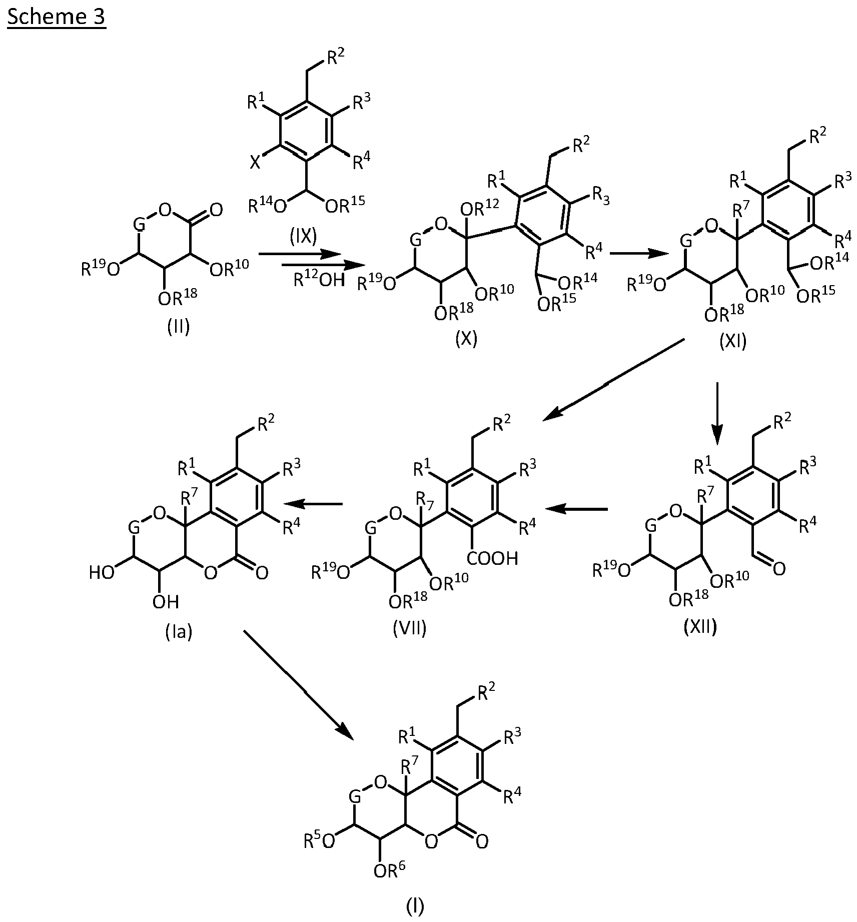

The key intermediate (X) can be prepared by means of a nucleophilic addition of (IX) (wherein X is selected from Br and I, and R¹⁴ and R¹⁵ may be short alkyl chains such as methyl, ethyl, or propyl, or R¹⁴ and R¹⁵ together may form an alkanediyl chain such as ethane-1,2-diyl, propane-1,3-diyl, or butane-1,4-diyl) to the lactone (II), followed by treatment with a compound of formula R¹²OH, wherein R¹² is a hydrogen atom or a methyl group, i.e. treatment with water or methanol in the presence of an acid such as ammonium chloride, methanesulfonic acid, toluenesulfonic acid, sulfuric acid, or hydrochloric acid to yield the intermediate (IV)

When R¹⁰, R¹⁸ and R¹⁹ are hydroxyl protecting groups such as benzyl, methyl, ethyl, methoxymethyl, 2-methoxyethoxymethyl, trialkylsilyl, and the like; the reaction may be carried out in the presence of a lithium donor, for example, *n-, sec-,* or *tert*-butyl lithium. These reactions are preferably conducted between -100 and 0 ºC, particularly preferably between -80 and -10 ºC, in an inert solvent or mixtures thereof, such as tetrahydrofuran, diethyl ether, toluene, heptane, or dichloromethane.

When R¹⁰, R¹⁸ and R¹⁹ are hydroxyl protecting groups such as acyl (preferably acetyl or benzoyl), alkoxycarbonyl, but also benzyl, methyl, ethyl, and the like, the reactions may be carried out in the presence of a Grignard reagent such as isopropylmagnesium bromide or diisopropylmagnesium. These reactions are preferably conducted between -80 and 40 ºC, particularly preferably between 0 and 20 ºC, in an inert solvent or mixtures thereof, such as tetrahydrofuran, diethyl ether, toluene, heptane, or dichloromethane.

The C-glycoside (XI), when R⁷ is -H, is obtained by a reductive removal of the resulting lactol or ketal (X), coming from the previous reaction, with a reducing agent in the presence of a Lewis or Brönsted acid. Suitable reducing agents include silanes such as triethyl-, triisopropyl-, or triphenylsilane, boranes, lithium aluminum hydride, diisobutylaluminum hydride, sodium borohydride, or sodium cyanoborohydride. The reactions are performed without or in the presence of a Lewis acid such as boron trifluoride etherate, tin tetrachloride, titanium tetrachloride, trimethylsilyltriflate, or zinc iodide, or a Brönsted acid such as hydrochloric acid, acetic acid, or trifluoroacetic acid. The reductions are carried out in a solvent or mixtures thereof, such as dichloromethane, chloroform, acetonitrile, dioxane, tetrahydrofuran, hexane, toluene, ethanol, or water at temperatures between -80 and 30 ºC.

The acid cleavage of the ketal moiety of (XI) by treatment with toluenesulfonic acid, acetic acid, trifluoroacetic acid, hydrochloric acid, sulfuric acid, and the like, in a suitable solvent such as methanol, tetrahydrofuran, ethyl acetate, water, and the like, leads to the aldehyde (XII).

The subsequent oxidation of (XII) by treatment with NaClO, potassium permanganate, hydrogen peroxide, and the like, affords the carboxylic acid (VII). The reactions are preferably conducted between -20 and 60 ºC, more preferably between 0 and 40 ºC, in a suitable solvent such as water, acetone, dioxane, tert-butanol, tetrahydrofuran, or dimethylformamide.

Alternatively, the reaction between (XI) and an oxidant such as NaClO, LiClO, potassium permanganate, and the like, in the presence of an acid such as aminosulfonic acid, sulfuric acid, and the like, allows the direct preparation of (VII). The reactions are conducted preferably between -20 and 60 ºC, more preferably between 0 and 30 ºC, in a suitable solvent such as dichloromethane, tetrahydrofuran, acetone, acetonitrile, dioxane, and the like.

The subsequent lactonization of (VII) can be conducted as described in Scheme 1.

Alternatively, the synthesis of the key intermediate (XI) wherein R⁷ is a hydrogen atom can be achieved using the methodology depicted in Scheme 4.

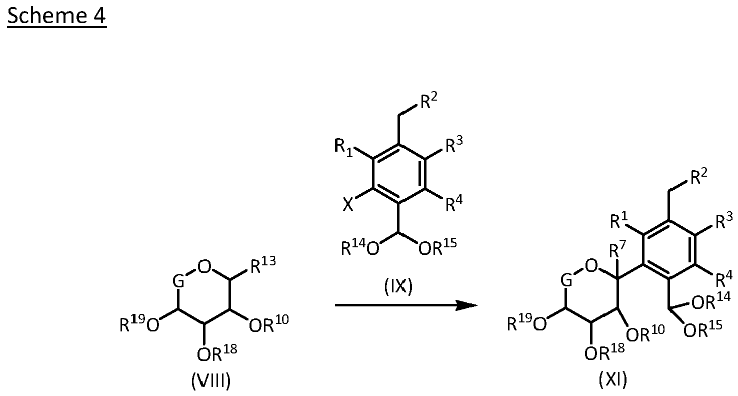

The key intermediate (XI) can be prepared by reaction of the aryl halide (IX), (wherein X is Cl, Br, I, ZnCl, ZnBr, or Znl, and R¹⁴ and R¹⁵ may be short alkyl chains such as methyl, ethyl, or propyl, or R¹⁴ and R¹⁵ together may form an alkanediyl chain such as ethane-1,2-diyl, propane-1,3-diyl, or butane-1,4-diyl), and a conveniently substituted compound of formula (VIII), wherein R¹³ can be CI, Br, I, Ac, hydroxyl, methoxy, trichloroacetamide, or any other good leaving group, and R¹⁰, R¹⁸ and R¹⁹ are hydroxyl protecting groups such as benzyl, methyl, ethyl, methoxymethyl, trialkylsilyl, acyl (preferably acetyl or benzoyl), alkoxycarbonyl, and the like. Thus, when X is Cl, Br, or I the reaction takes place in the presence of Mg in a solvent such as diethyl ether or tetrahydrofuran. On the other hand, when X is ZnCl, ZnBr, or Znl a Negishi cross-coupling reaction can be conducted in the presence of an Ni catalyst such as NiCl₂, NiBr₂, or Ni(COD)₂ and a suitable ligand such as diglyme or 2,6-bis[(4*R*)-4-phenyl-2-oxazolinyl]pyridine (PyBox) using a solvent such dimethylformamide, dimethylacetamide, 1,3-dimethyl-2-imidazolidinone, or tetrahydrofuran [see Gong, H., Gagné, M.R. J Am Chem Soc 2008, 130(36): 12177-83].

In Scheme 5, a third strategy which allows the preparation of the above discussed intermediate (V) wherein R⁷ is a hydroxyl group is shown.

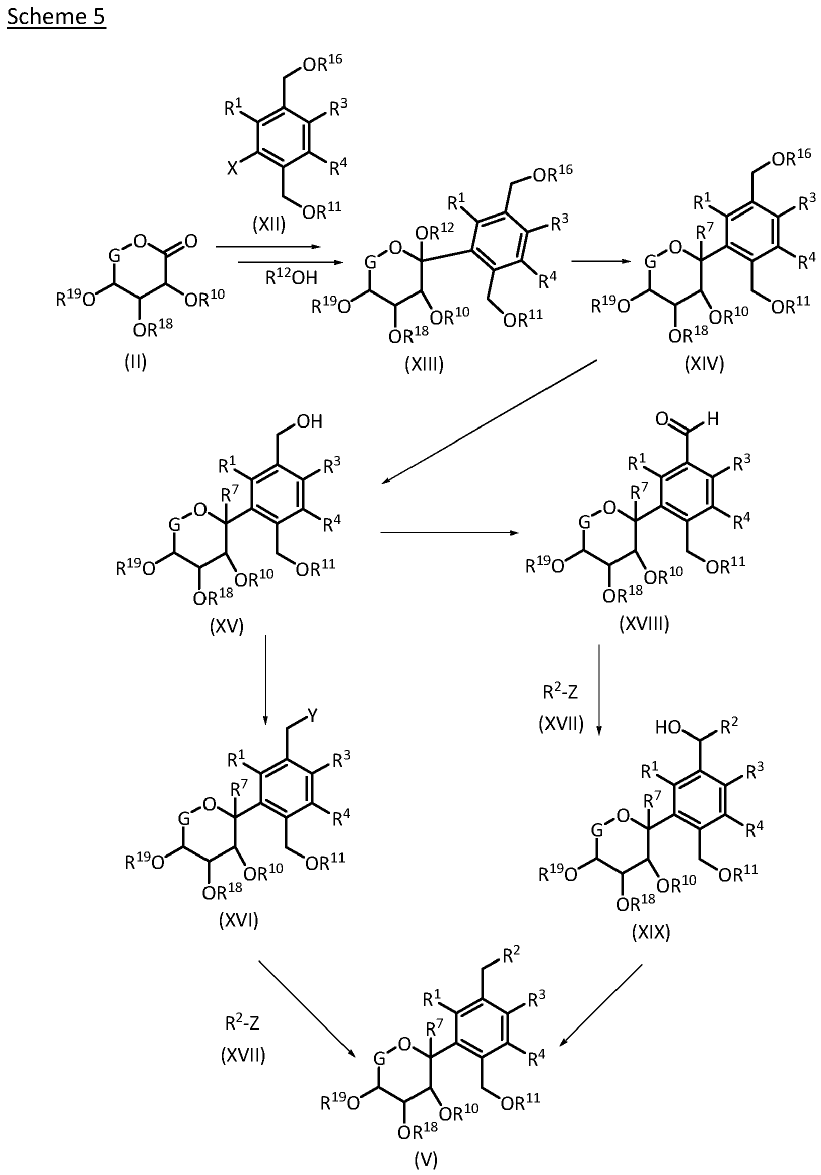

This strategy indicates that the key intermediate (XIV) can be prepared by means of a two-step C-glycosidation between lactone (II) and the aromatic halide (XII) (wherein R¹⁶ is a hydroxyl protecting group such as trialkylsilyl, methoxymethyl, 2-methoxyethoxymethyl, triphenylmethyl, and the like) followed by treatment with a compound of formula R¹²OH, wherein R¹² is a hydrogen atom or a methyl group, i.e. treatment with water or methanol in the presence of an acid such as ammonium chloride, methanesulfonic acid, toluenesulfonic acid, sulfuric acid, or hydrochloric acid to yield the intermediate (IV). The synthetic methodology is the same as discussed above in Scheme 1. The cleavage of the protecting group R¹⁶ following the methodologies previously discussed leads to the benzyl alcohol (XV). The conversion of the hydroxyl group of (XV) into a good leaving group Y in (XVI) such as Cl, Br, I, mesylate, tosylate, and the like, is achieved following standard strategies known to those skilled in the art. The displacement of this leaving group is carried out with the species (XVII) (wherein Z is Cl, Br, or I) to yield (V). The reaction is performed in the presence of a metal donor such as a lithium donor, for example, *n-, sec-,* or *tert*-butyl lithium, or a Grignard reagent such as isopropylmagnesium bromide or diisopropylmagnesium. The former reactions are preferably conducted between -100 and 0 ºC, particularly preferably between -80 and -10 ºC, in an inert solvent or mixtures thereof, such as tetrahydrofuran, diethyl ether, toluene, heptane, or dichloromethane. The latter reactions are preferably conducted between -80 and 40 ºC, particularly preferably between 0 and 20 ºC, in an inert solvent or mixtures thereof, such as tetrahydrofuran, diethyl ether, toluene, heptane, or dichloromethane.

Alternatively, the benzyl alcohol (XV) can be oxidized to the aldehyde (XVIII) using Dess-Martin periodane in dichloromethane, Swern oxidation conditions (oxalyl chloride, dimethyl sulfoxide, trialkylamine), manganese (IV) oxide in dichloromethane, pyridinium chlorochromate in dichloromethane, and the like. The aldehyde (XVIII) in turn can be reacted with (XVII) using similar conditions to those discussed above to afford the alcohol (XIX). Finally, the reduction of the latter with triethylsilane in the presence of a suitable Lewis acid such as boron trifluoride etherate, tin tetrachloride, and the like, lithium aluminum tetrahydride or sodium tetrahydroborate in the presence of aluminum trichloride in diethyl ether, tetrahydrofuran, and the like, or hydrogen in the presence of a suitable catalyst such as Pd/C, Pd(OH)₂, PdCl₂, and the like, leads to (V).

On the other hand, the synthesis of the key intermediate (XIV) wherein R⁷ is a hydrogen atom can be achieved using the methodology depicted in Scheme 6.

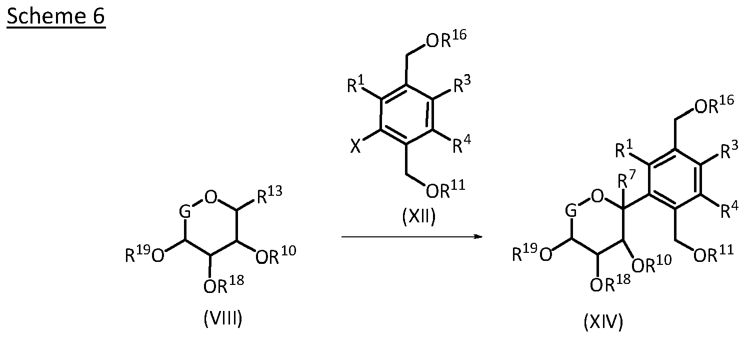

The followed strategy of coupling the species (VIII) and (XII) is similar to that described above in Scheme 2.

The synthesis of the intermediate aldehyde (XVIII) can also be achieved following the strategy depicted in Scheme 7.

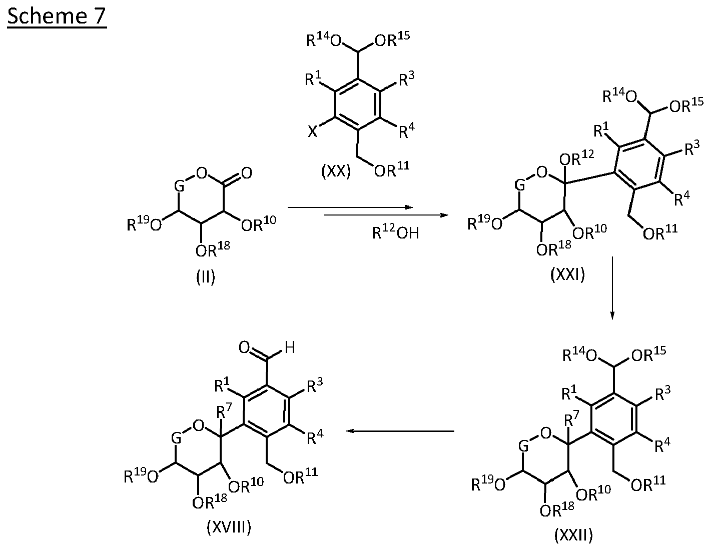

All the reactions are carried out in the same way as discussed hereinbefore.

Finally, the key intermediate (III) which appears in Scheme 1 can be prepared following the synthetic pathway depicted in Scheme 8.

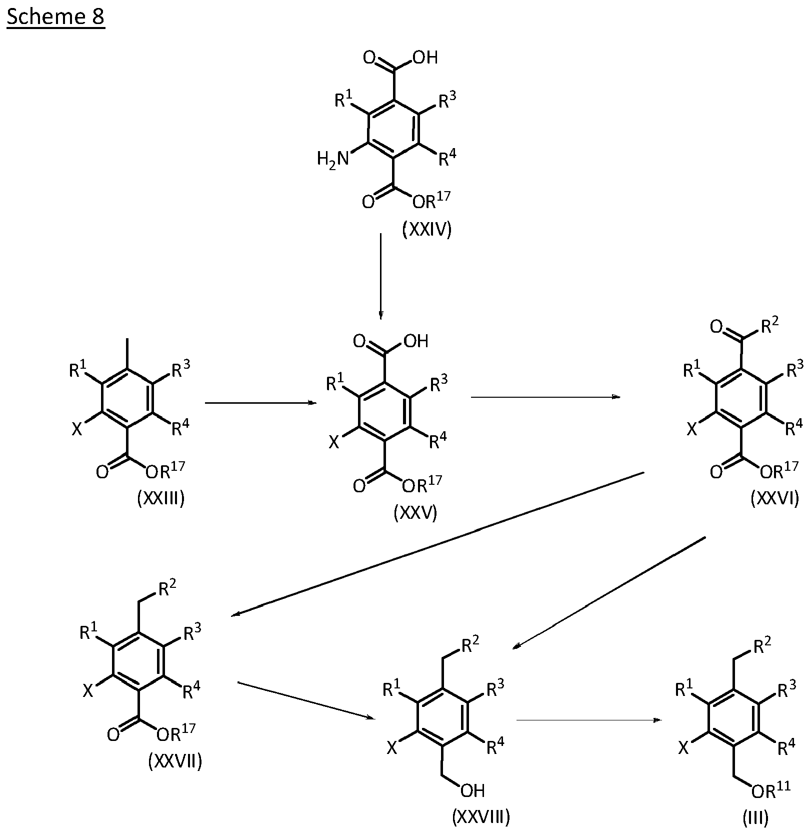

This strategy starts with the selective oxidation of the methyl group present in the substituted toluene derivative (XXIII) (wherein R¹⁷ can be short alkyl chains such as methyl, ethyl, or propyl). This transformation can be accomplished by reacting the derivative (XXIII) with an oxidant agent in a controlled medium. Suitable oxidant agents include CrO₃, K₂CrO₄, KMnO₄, or RuO₄. Reactions are conducted in a solvent or mixtures thereof such as acetonitrile, dichloroethane, dichloromethane, or water with a phase transfer catalyst such as 18-crown-6 ether or tetrabutylammonium salts (bromide, iodide, or chloride) at temperatures between 10 and 30 ºC and controlled pH (preferably between 8 and 10).

Compound (XXV) can also be obtained starting from compound (XXIV) using the Sandmeyer reaction conditions.

Treatment of the acid (XXV) with an excess of (COCl)₂ or SOCl₂ and a catalytic amount of dimethylformamide leads to the corresponding acyl chloride intermediate, which reacts with a properly substituted ring (R²) in the presence of a Lewis acid such as FeCl₃ or AlCl₃ to give the ketone (XXVI).

The ketone (XXVI) is reduced to the compound (XXVII) with a reducing agent in the presence of a Lewis or Brönsted acid. Suitable reducing agents include silanes such as triethyl-, triisopropyl-, or triphenylsilane, boranes, hydrogen, lithium aluminum hydride, diisobutylaluminum hydride, sodium borohydride or sodium cyanoborohydride. The reactions are performed without or in the presence of a Lewis acid such as boron trifluoride ethearate, tin tetrachloride, titanium tetrachloride, trimethylsilyltriflate, or zinc iodide, or a Brönsted acid such as hydrochloric acid, acetic acid, or trifluoroacetic acid. Reactions are carried out in a solvent or mixtures thereof, such as dichloromethane, chloroform, acetonitrile, dioxane, tetrahydrofuran, toluene, ethanol, or water, at temperatures between -80 and 30 ºC.

Further reduction of compound (XXVII) affords the alcohol (XXVIII). Suitable reducing agents include lithium aluminum hydride, diisobutylaluminum hydride or sodium, or lithium borohydride. Reactions are carried out in a solvent or mixtures thereof, such as dioxane, tetrahydrofuran, toluene, hexane, methanol, ethanol, or water, at temperatures between -40 and 30 ºC.

Intermediate (XXVIII) can be obtained directly from the ketone (XXVI) when a reducing agent such as sodium borohydride or sodium or lithium aluminum hydride is used in presence of AlCl₃ [see Nystrom, R.F., Berger, C.R.A. J Am Chem Soc 1958, 80(11): 2896-8].

The alcohol (XXVIII) is adequately protected to the intermediate ether (III), wherein R¹¹ is preferably allyl, by deprotonation and reaction with an adequate halide (chloro, bromo, or iodide) in the presence or not of a transfer catalyst. Suitable deprotonating agents include potassium hydroxide, sodium hydroxide, sodium hydride, and the like. Protection is carried out in a solvent such as tetrahydrofuran, dimethylformamide, toluene, or water in the presence or not of a transfer catalyst such as tetrabutylammonium salts (chlorine, bromide, or iodide) at temperatures between -10 and 30 ºC.

The following abbreviations are employed herein:
- DCM = dichloromethane
- DMF = dimethylformamide
- Et₂O = diethyl ether
- EtOAc = ethyl acetate
- g = gram(s)
- LAH = lithium aluminum hydride
- mg = milligram(s)
- mL = milliliter
- mmol = millimole(s)
- mol = mole(s)
- NEt₃ = triethylamine
- THF = tetrahydrofuran

The term "alkyl" as employed herein alone or as part of another group designates both straight- and branched-chain saturated hydrocarbons containing 1 to 20 carbons, preferably 1 to 8 carbons, and more preferably 1 to 4 carbons. Examples of alkyl groups are methyl, ethyl, *n*-propyl, isopropyl, *n*-butyl, isobutyl, *tert*-butyl, pentyl, hexyl, isohexyl, heptyl, 4,4-dimethylpentyl, octyl, 2,2,4-trimethylpentyl, nonyl, decyl, undecyl, dodecyl, and the various branched-chain isomers thereof.

The term "alkylidene" as employed herein alone or as part of another group refers to a divalent radical of an alkyl group as described above obtained by removal of two hydrogen atoms from the same carbon atom, the free valences of which are part of a double bond, containing 1 to 20 carbons, preferably 1 to 8 carbons, and more preferably 1 to 4 carbons. Examples of alkylidene groups are methylidene, ethylidene, propylidene, and butylidene, and the various branched-chain isomers thereof.

The term "alkenyl" as employed herein alone or as part of another group includes both straight- and branched-chain hydrocarbons containing 2 to 20 carbons, preferably 2 to 8 carbons, and more preferably 2 to 4 carbons, and includes 1 to 6 double bonds. Examples of alkenyl groups are vinyl, allyl, 2-propenyl, 2-butenyl, 3-butenyl, 3-pentenyl, 4-pentenyl, 2-hexenyl, 3-hexenyl, 2-heptenyl, 4-heptenyl, 3-octenyl, 3-nonenyl, 4-decenyl, 3-undecenyl, and 4,8,12-tetradecatrienyl.

The term "alkynyl" as employed herein alone or as part of another group includes both straight- and branched-chain hydrocarbons containing from 2 to 20 carbons, preferably 2 to 8 carbons, and more preferably 2 to 4 carbons, and includes 1 to 6 triple bonds and optionally 1 to 3 double bonds. Examples of alkynyl groups are 2-propynyl, 3-butynyl, 4-pentynyl, 2-hexynyl, 4-heptynyl, 3-octynyl, 3-nonynyl, 4-decynyl, 3-undecynyl, and 4-dodecynyl.

The term "alkyloxy" or "alkoxy" as employed herein alone or as part of another group designates a group consisting of an alkyl group as defined above bonded to an oxygen atom. Examples of alkoxy groups are methoxy, ethoxy, *n*-propoxy, isopropoxy, *n-*butoxy, isobutoxy, *tert*-butoxy, pentoxy, hexoxy, isohexoxy, heptoxy, 4,4-dimethylpentoxy, octoxy, and 2,2,4-trimethylpentoxy.

The term "cycloalkyl" as employed herein alone or as part of another group includes saturated cyclic hydrocarbon groups containing 1 to 3 rings, including monocyclic alkyl, bicyclic alkyl, and tricyclic alkyl systems, and containing a total of 3 to 20 carbon atoms, preferably 3 to 10 carbons, and more preferably 3 to 6 carbons, forming part of the ring system. Examples of cycloalkyl groups are cyclopropyl, cyclobutyl, cyclopentyl, cyclohexyl, cycloheptyl, cyclooctyl, cyclodecyl, cyclododecyl, adamantyl , and bicyclo[3.3.3]undecanyl.

The term "cycloalkylidene" as employed herein alone or as part of another group refers to a divalent radical of a cycloalkyl group as described above, obtained by removal of two hydrogen atoms from the same carbon atom, the free valences of which are part of a double bond, containing 3 to 20 carbons, preferably 3 to 10 carbons, and more preferably 3 to 6 carbons. Examples of cycloalkylidene groups are cyclopropylidene, cyclobutylidene, cyclopentylidene, and cyclohexylidene.

The term "aryl" as employed herein alone or as part of another group refers to monocyclic or bicyclic aromatic groups containing 6 to 10 carbons in the ring portion, such as phenyl or naphthyl (including 1-naphthyl and 2-naphthyl), and may optionally include 1 to 3 additional fused carbocyclic rings, such as cycloalkyl. Examples of aryl groups are phenyl, 1-naphthyl, 2-naphthyl, and tetrahydronaphthyl.

The term "heterocyclyl" as employed herein alone or as part of another group refers to a 5-, 6-, or 7-membered saturated or partially unsaturated ring which includes 1 to 2 heteroatoms, selected from the group consisting of nitrogen, oxygen, and sulfur, and such rings optionally fused to an aryl, cycloalkyl, heteroaryl, or heterocyclyl ring. The heterocyclyl group is linked through a carbon atom or a heteroatom.

The term "heteroaryl" as employed herein alone or as part of another group refers to a 5- or 6-membered aromatic ring which includes 1, 2, 3, or 4 heteroatoms, selected from the group consisting of nitrogen, oxygen, and sulfur, and to such rings optionally fused to an aryl, cycloalkyl, heteroaryl, or heterocyclyl ring, i.e., a "monocyclic heteroaryl" refers to a heteroaryl as defined above without any fused ring; and a "bicyclic heteroaryl" as employed herein refers to an 8-, 9-, or 10-membered two-ring system which includes 1, 2, 3, or 4 heteroatoms selected from the group consisting of nitrogen, oxygen, and sulfur, wherein at least one of the rings is a heteroaryl, as previously defined.

The term "halogen" as used herein alone or as part of another group refers to chlorine, bromine, fluorine, and iodine.

The term "hydroxyl" as used herein alone or as part of another group refers to -OH.

In the context of the present invention, when a group is said to be polysubstituted it is to be understood that 2 or more hydrogen atoms in said group are replaced by 2 or more substituents, preferably by 2 to 12 substituents, more preferably by 2 to 10 substituents, more preferably by 2 to 6 substituents, and even more preferably by 2 to 4 substituents.

As used herein, the term "pharmaceutically acceptable salt" embraces salts with a pharmaceutically acceptable acid or base, which are synthesized from the parent compound which contains an acidic moiety by addition of a pharmaceutically acceptable base, or which are synthesized from the parent compound which contains a basic moiety by addition of a pharmaceutically acceptable acid. Pharmaceutically acceptable acids include both inorganic acids, for example, hydrochloric, sulfuric, phosphoric, diphosphoric, hydrobromic, hydroiodic, and nitric acid, and organic acids, for example, citric, fumaric, maleic, malic, mandelic, ascorbic, oxalic, succinic, tartaric, benzoic, acetic, methanesulfonic, ethanesulfonic, benzenesulfonic, or *p-*toluenesulfonic acid. Pharmaceutically acceptable bases include alkali metal (e.g., sodium or potassium) and alkali earth metal (e.g., calcium or magnesium) hydroxides and organic bases, such as alkyl amines, arylalkyl amines, and heterocyclic amines.

All stereoisomers of the compounds of this invention are contemplated either alone or as mixtures thereof. The process of preparation can utilize racemates, enantiomers, or diastereomers as starting materials. When diastereomeric or enantiomeric products are prepared, they can be separated by conventional methods, for example, chromatographic or functional crystallization.

The term "treatment" or "treating" in the context of this document refers to administration of a compound or a formulation according to this invention to prevent, improve, or eliminate the disease or one or more symptoms associated with the disease. "Treatment" also encompasses preventing, improving, or eliminating the physiological sequelae of the disease.

The term "pharmaceutically suitable excipient" refers to a vehicle, diluent, or adjuvant that is administered with the active ingredient. Such pharmaceutical excipients can be sterile liquids, such as water and oils, including those of petroleum, animal, vegetable, or synthetic origin, such as peanut oil, soybean oil, mineral oil, sesame oil, and similars. Water or saline aqueous solutions and aqueous dextrose and glycerol solutions, particularly for injectable solutions, are preferably used as vehicles. Suitable pharmaceutical vehicles are described in "Remington's Pharmaceutical Sciences" by E.W. Martin, 21st Edition, 2005; or the "Handbook of Pharmaceutical Excipients" by C.R. Rowe, J.S. Paul, E.Q. Marian, 6th Edition.

Drugs of the present invention may be administered orally, parenterally, subcutaneously, rectally, topically, by inhalation, and locally. Any administration method commonly used for drugs, such as tablets, coated tablets, capsules, solution (including nanoemulsion), syrup, powder, suppository, cream, and ointment, may be used. The pharmaceutical composition can be formulated employing conventional liquid or solid vehicles or diluents and pharmaceutical additives, according to the desired mode of administration. The mentioned formulations will be prepared using standard methods, such as those described or referred to in the Spanish and U.S. Pharmacopoeias and similar reference texts.

### EXAMPLES

The following examples represent specific embodiments of the present invention. They do not intend to limit in any way the scope of the invention defined in the present description.

### Example 1

### (2R,3S,4S,4aR,10bS)-9-(4-Ethylbenzyl)-3,4-dihydroxy-2-(hydroxymethyl)-3,4,4a,10b-tetrahydropyrano[3,2-c][2]benzopyran-6(2H)-one

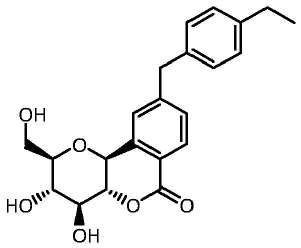

### 3-Bromo-4-methoxycarbonylbenzoic acid

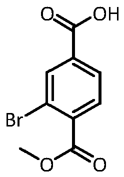

### Path A [Sasson, Y. et al. J Org Chem 1985, 51(15): 2880-3]

In a round-bottom flask, 2-bromo-4-methylbenzoic acid methyl ester (10.00 g, 42.8 mmol), tetrabutyl ammonium bromide (345 mg, 0.86 mmol), ruthenium chloride trihydrate (86 mg, 0.43 mmol), dichloroethane (14 mL) and sodium hypochlorite 10% (115 mL) were mixed. The pH at the aqueous phase was adjusted to 9.0 by addition of 20% v/v H₂SO₄. The biphasic mixture was stirred for 2.5 hours, and meanwhile, NaOH 20% v/v was added to keep the pH at 9. The phases were separated and the aqueous phase was washed with DCM (3 × 50 mL), acidified with 20% H₂SO₄ and extracted with EtOAc (3 × 50 mL). The organic layer was washed with brine (50 mL), dried over anhydrous magnesium sulfate, filtered and evaporated to dryness in vacuo to afford 3-bromo-4-methoxycarbonylbenzoic acid as a whitish solid (2.33 g, 21% yield). ¹H-NMR (400 MHz, CDCl₃): 8.38 (d, *J* = 1.5, 1H), 8.07 (dd, *J* = 8.1, 1.6, 1H), 7.84 (d, *J* = 8.1, 1H), 3.98 (s, 3H).

### Path B [WO 2004/108683]

In a round-bottom flask, 3-amino-4-methoxycarbonylbenzoic acid (1.95 g, 10 mmol) was suspended in a mixture of 47% HBr (50 mL) and acetic acid (50 mL). The suspension was cooled to -10 ºC, an aqueous solution (20 mL) of sodium nitrite (0.69 g, 10 mmol) was added and the mixture was stirred for 30 minutes. The resulting solution was added to a solution of copper bromide (1.44 g, 10 mmol) in 47% HBr (50 mL) at -10 ºC and then warmed to room temperature for 1 hour. The mixture was diluted with EtOAc (100 mL) and washed with water (5 × 50 mL). The organic phase was dried over anhydrous magnesium sulfate and filtered to afford 1.76 g of crude product, which was purified by silica gel column chromatography to yield the desired product as a yellowish solid (1.40 g, 55 % yield).

When the reaction was scaled up the procedure was changed to concentrate solvents as follows:
In a round-bottom flask equipped with magnetic stirrer and addition funnel, 3-amino-4-methoxycarbonylbenzoic acid (20.07 g, 99.75 mmol) was suspended in a mixture of 47% HBr (40 mL) and acetic acid (70 mL). The suspension was cooled to 0 ºC and water (40 mL) was added. The mixture was cooled to -10 ºC and an aqueous solution (20 mL) of sodium nitrite (7.00 g, 100 mmol) was added and the mixture was stirred at 0 ºC for 2 hours. The resulting suspension was added to a solution of copper bromide (14.50 g, 100 mmol) in 47% HBr (50 mL) and heated at 60 ºC for 1 hour. The reaction mixture was poured into ice/water (500 g) and the precipitated solid was filtered and washed with water (3 × 200 mL). The filtrate was dissolved with EtOAc, dried over anhydrous magnesium sulfate and filtered to afford the desired product as a yellowish solid (24.95 g, 96 % yield).

### 2-Bromo-4-(4-ethylbenzoyl)benzoic acid methyl ester [Lv, B. et al. Bioorg Med Chem Lett 2009, 19(24): 6877-81]

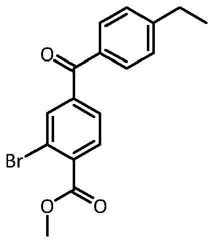

In a round-bottom flask, 3-bromo-4-methoxycarbonylbenzoic acid (5.03 g, 17.5 mmol) was dissolved in DCM (150 mL). Thionyl chloride (2.3 mL, 31.3 mmol) and dimethylformamide (0.1 mL) were added and the resulting suspension was heated at 50 ºC for 1 hour. The resulting solution was evaporated to dryness in vacuo, affording an oily residue that was dissolved in DCM (150 mL) under inert atmosphere and ethylbenzene (2.6 mL, 21.02 mmol) was added. After cooling to 0 ºC with an ice bath, AlCl₃ (5.30 g, 46.1 mmol) was added portionwise. The resulting suspension was heated at 50 ºC for 1.5 hours and then poured into ice/water (400 g). The phases were separated and the aqueous layer extracted with DCM (4 × 100 mL). The organic layer was washed with brine (100 mL), dried over anhydrous magnesium sulfate, filtered and evaporated to dryness in vacuo to afford 6.30 g of the title compound, which was purified by silica gel column chromatography to afford 2-bromo-4-(4-ethylbenzoyl)benzoic acid methyl ester as an oil (4.61 g, 76% yield).

### 2-Bromo-4-(4-ethylbenzyl)benzoic acid methyl ester

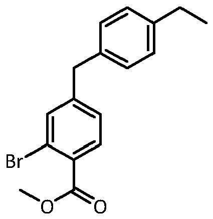

In a round-bottom flask equipped with magnetic stirring and inert atmosphere, 2-bromo-4-(4-ethylbenzoyl)benzoic acid methyl ester (4.61 g, 13.3 mmol) was dissolved in trifluoroacetic acid (24 mL). Firstly, triethylsilane (4.1 mL, 25.41 mmol) was added at room temperature and then over 10 minutes trifluoromethanesulfonic acid (73 µL, 0.81 mmol) was added. The resulting mixture was heated at 75 ºC for 2 hours and then evaporated to dryness in vacuo. The residue was dissolved in EtOAc (300 mL) and washed with water (3 × 100 mL), 10% aqueous sodium hydrogen carbonate (3 × 100 mL) and brine (3 × 100 mL). The organic phase was dried over anhydrous magnesium sulfate, filtered and evaporated to dryness in vacuo to give 7.92 g of a residue, which was purified by silica gel column chromatography to afford 2-bromo-4-(4-ethylbenzyl)benzoic acid methyl ester (3.51 g, 79% yield) as a colorless oil.

### 2-Bromo-4-(4-ethylbenzyl)benzenemethanol

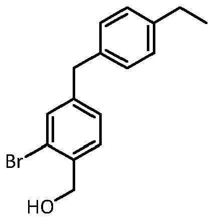

In a round-bottom flask equipped with magnetic stirring and inert atmosphere, 2-bromo-4-(4-ethylbenzyl)benzoic acid methyl ester (3.40 g, 10.35 mmol) was dissolved in anhydrous THF (60 mL). The resulting solution was cooled to 0 ºC with an ice bath and LAH (1.62 g, 41.39 mmol) was added portionwise. The mixture was allowed to warm to room temperature and stirred for 3 hours. Water (1.6 mL), 15% aqueous NaOH (1.6 mL) and water (4.8 mL) were added dropwise sequentially. The mixture was stirred for 15 minutes and then filtered through a Celite pad and evaporated to dryness in vacuo to afford 2-bromo-4-(4-ethylbenzyl)benzenemethanol (3.10 g, 99% yield) as a white solid.

### 1-(Allyloxymethyl)-2-bromo-4-(4-ethylbenzyl)benzene

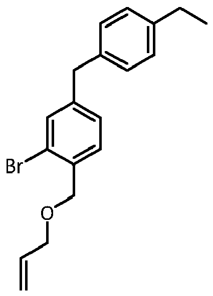

In a round-bottom flask equipped with magnetic stirring and inert atmosphere, 2-bromo-4-(4-ethylbenzyl)benzenemethanol (3.06 g, 10.0 mmol) was dissolved in anhydrous DMF (50 mL). The resulting solution was cooled to 0 ºC with an ice bath and NaH (882 mg, 22.0 mmol) was added portionwise. The mixture was stirred at this temperature for 30 minutes and afterwards tetrabutylammonium iodide (374 mg, 1.0 mmol) and allyl bromide (1.1 mL, 12.0 mmol) were added. The mixture was allowed to warm to room temperature and stirred overnight. Water (20 mL) and EtOAc (80 mL) were added and phases were separated. The aqueous layer was extracted with EtOAc (3 x 50 mL) and the collected combined organic fractions were washed with brine (5 × 50 mL), dried over anhydrous magnesium sulfate, filtered and evaporated to dryness in vacuo to afford 3.92 g of a residue, which was purified by silica gel column chromatography to afford methyl 1-(allyloxymethyl)-2-bromo-4-(4-ethylbenzyl)benzene (3.18 g, 92% yield) as a colorless oil.

### (2S,3S,4R,5R,6R)-2- [2-(Allyloxymethyl)-5-(4-ethylbenzyl)phenyl]-3,4,5-tris(benzyloxy)-6-(benzyloxymethyl)tetrahydro-2H-pyran

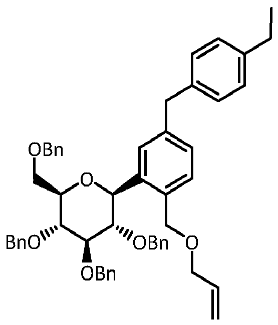

In a round-bottom flask equipped with magnetic stirring and inert atmosphere, methyl 1-(allyloxymethyl)-2-bromo-4-(4-ethylbenzyl)benzene (1.65 g, 4.78 mmol) was dissolved in anhydrous THF (29 mL) and cooled to -78 ºC. *n*-BuLi 1.6 M in hexane (3.1 mL, 4.96 mmol) was added dropwise and the reaction mixture was stirred for 15 minutes at -78 ºC. A freshly prepared solution of (3*R*,4*S*,5*R*,6*R*)-3,4,5-tris(benzyloxy)-6-(benzyloxymethyl)tetrahydro-2*H*-pyran-2-one (2.62 g, 4.86 mmol) in anhydrous THF (10 mL) under inert atmosphere was added dropwise at -78 ºC. The reaction mixture was stirred at this temperature during 1 hour and then allowed to warm to -5 ºC during 2 hours. A saturated ammonium chloride solution was added (23 mL) and the mixture was allowed to warm to room temperature during 30 minutes. After dilution with DCM (50 mL) the phases were separated and the aqueous layer was extracted with DCM (3 × 50 mL). The combined organic fractions were washed with brine (5 × 50 mL), dried over anhydrous magnesium sulfate, filtered and evaporated to dryness in vacuo to afford 3.92 g of an oily residue. The obtained residue was dissolved in DCM (140 mL) under inert atmosphere and cooled to -25 ºC. Et₃SiH (0.76 mL, 4.75 mmol) and BF₃·Et₂O (0.70 mL, 5.51 mmol) were added sequentially dropwise and then the resulting mixture was allowed to warm to 5 ºC during 2 hours. The mixture was quenched with 10% NaHCO₃ (40 mL) and the phases were separated. The aqueous layer was extracted with DCM (3 × 20 mL) and the combined organic fractions were washed with brine, dried over anhydrous magnesium sulfate, filtered and evaporated to dryness in vacuo to give 3.95 g of an oily residue, which was purified by silica gel column chromatography to afford (2*S*,3*S*,4*R*,5*R*,6*R*)-2-[2-(allyloxymethyl)-5-(4-ethylbenzyl)phenyl]-3,4,5-tris(benzyloxy)-6-(benzyloxymethyl)tetrahydro-2*H*-pyran (1.24 g, 32% yield) as a colorless oil.

### 4-(4-Ethylbenzyl)-2-[(2S,3S,4R,5R,6R)-3,4,5-tris(benzyloxy)-6-(benzyloxymethyl) tetrahydro-2H-pyran-2-yl]benzenemethanol

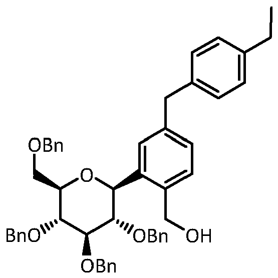

In a round-bottom flask equipped with magnetic stirring, (2*S*,3*S*,4*R*,5*R*,6*R*)-2-[2-(allyloxymethyl)-5-(4-ethylbenzyl)phenyl]-3,4,5-tris(benzyloxy)-6-(benzyloxymethyl) tetrahydro-2*H*-pyran (1.16 g, 1.47 mmol) was dissolved in a 9:1 mixture of acetic acid/water (20 mL) at room temperature. Sodium acetate (476 mg, 5.48 mmol) and PdCl₂ (566 mg, 3.20 mmol) were added and the resulting suspension was heated at 70 ºC for 90 minutes. Then, it was cooled to room temperature, diluted with EtOAc and filtered through a Celite pad. The filtrate was evaporated to dryness in vacuo giving an oily residue, which was purified by silica gel column chromatography to afford 4-(4-ethylbenzyl)-2-[(2*S*,3*S*,4*R*,5*R*,6*R*)-3,4,5-tris(benzyloxy)-6-(benzyloxymethyl)tetrahydro-2*H*-pyran-2-yl]benzenemethanol (828 mg, 75% yield) as a colorless oil.

### 4-(4-Ethylbenzyl)-2-[(2S,3S,4R,5R,6R)-3,4,5-tris(benzyloxy)-6-(benzyloxymethyl) tetrahydro-2H-pyran-2-yl]benzoic acid

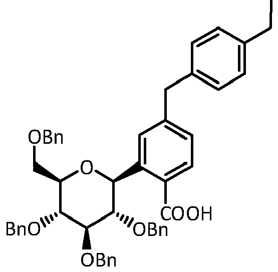

In a round-bottom flask equipped with magnetic stirring, 4-(4-ethylbenzyl)-2-[(2*S*,3*S*,4*R*,5*R*,6*R*)-3,4,5-tris(benzyloxy)-6-(benzyloxymethyl)tetrahydro-2*H*-pyran-2-yl]benzenemethanol (806 mg, 1.08 mmol) was dissolved in acetone (11 mL) at room temperature and then cooled to 0 ºC with an ice bath. 2.9 M Jones Reagent solution (0.74 mL, 2.15 mmol) was added dropwise and allowed to warm to room temperature. After 2.5 hours, the blue-green suspension was cooled again and an additional 2.9 M Jones Reagent solution (0.13 mL) was added dropwise and stirred for 4 hours at room temperature. Afterwards, it was diluted with EtOAc (75 mL) and extracted with a saturated aqueous solution of K₂S₂O₃ (3 × 20 mL). The organic fraction was dried over anhydrous magnesium sulfate, filtered and evaporated to dryness in vacuo to afford 4-(4-ethylbenzyl)-2-[(2*S*,3*S*,4*R*,5*R*,6*R*)-3,4,5-tris(benzyloxy)-6-(benzyloxymethyl)tetrahydro-2*H*-pyran-2-yl]benzoic acid (795 mg, 97% yield) as a colorless oil, which was used in the next step without further purification.

### (2R,3S,4S,4aR,10bS)-9-(4-Ethylbenzyl)-3,4-dihydroxy-2-(hydroxymethyl)-3,4,4a,10b-tetrahydropyrano[3,2-c][21]benzopyran-6(2H)-one

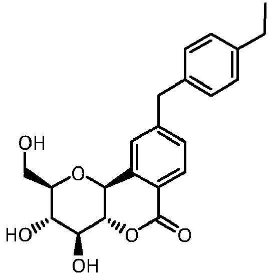

A solution of 4-(4-ethylbenzyl)-2-[(2*S*,3*S*,4*R*,5*R*,6*R*)-3,4,5-tris(benzyloxy)-6-(benzyloxymethyl)tetrahydro-2*H*-pyran-2-yl]benzoic acid (795 mg, 1.04 mmol) in EtOAc (20 mL) and MeOH (60 mL) was placed in a pressurized reactor. PdCl₂ (117 mg) was added to the solution and the reactor was purged with vacuum and nitrogen. Afterwards, hydrogen was charged up to 3 bar, stirring was continued for 16 hours, and finally, the system was purged again. The suspension was filtered through a Celite pad, washed with methanol and the filtrate was evaporated to dryness under vacuum to afford 450 mg of a whitish solid, which was purified by silica gel column chromatography to afford the title compound as a whitish solid (278 mg, 69% yield).

### Example 2

### (2R,3S,4S,4aR,10bS)-8-Chloro-9-(4-ethoxybenzyl)-3,4-dihydroxy-2-(hydroxymethyl)-3,4,4a,10b-tetrahydropyrano[3,2-c][2]benzopyran-6(2H)-one

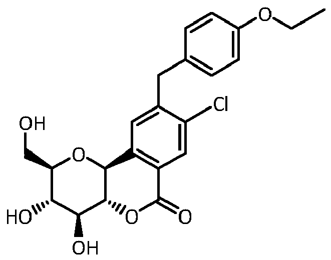

### 5-Amino-2-bromo-4-methylbenzoic acid

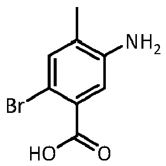

In a round-bottom flask equipped with magnetic stirring and inert atmosphere, 3-amino-4-methylbenzoic acid (24.72 g, 162 mmol) was dissolved in anhydrous DMF (140 mL). The solution was then cooled to 0 - 5ºC and *N*-bromosuccinimide (29.70 g, 163.5 mmol) was added portionwise at a rate that the reaction mixture was kept below 15 ºC. The mixture was reacted for 2 hours and then poured into ice/water (150 g) with stirring. The produced solid was filtered and washed with cool water (3 x 120 mL). The filter cake was dissolved with EtOAc (800 mL) and washed with water (3 × 200 mL). The organic layer was dried over anhydrous magnesium sulfate, filtered and evaporated to dryness in vacuo to afford 5-amino-2-bromo-4-methylbenzoic acid as a pink solid (31.59 g, 85% yield).

### 5-Amino-2-bromo-4-methylbenzoic acid methyl ester

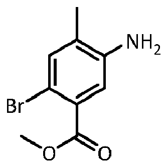

In a round-bottom flask equipped with magnetic stirring and a reflux condenser with a calcium chloride tube, 5-amino-2-bromo-4-methylbenzoic acid (39.90 g, 0.17mol) was suspended in methanol (500 mL). The suspension was cooled to 0 to 5 ºC and thionyl chloride (40 mL, 0.54 mol) was added dropwise. The mixture was heated to reflux for 6 hours. The resulting solution was concentrated in vacuo and the residue was diluted with ice/water (500 g). The solution was neutralized to pH 7.5 with saturated aqueous NaHCO₃ and extracted with EtOAc (3 x 200 mL). The collected combined organic phases were washed with brine (3 x 200 mL), dried over anhydrous magnesium sulfate, filtered and evaporated to dryness in vacuo to afford 5-amino-2-bromo-4-methylbenzoic acid methyl ester as a whitish solid (40.35 g, 95% yield).

### 2-Bromo-5-chloro-4-methylbenzoic acid methyl ester [WO 2004/108683]

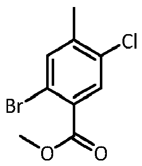

In a round-bottom flask equipped with magnetic stirring and addition funnel, 5-amino-2-bromo-4-methylbenzoic acid methyl ester (29.70 g, 121.7 mmol) was dissolved in dioxane (150 mL). The solution was cooled to 15 ºC and HCl 37% (137 mL) was added dropwise. The mixture was cooled to -10 ºC and an aqueous solution (21 mL) of sodium nitrite (8.91 g, 127 mmol) was added dropwise at a rate such that the reaction temperature was kept below 0 ºC. After being stirred at this temperature for 2 hours, the resulting suspension was added to a solution of copper chloride (14.47 g, 145 mmol) in 37% HCl (69 mL). The mixture was stirred at room temperature for 40 minutes and then poured into ice/water (650 g). The precipitated solid was filtered in vacuo and washed with water (3 × 200 mL). The filtrate was dissolved with EtOAc (300 mL) and washed with brine (2 × 100 mL). The organic layer was dried over anhydrous magnesium sulfate, filtered and evaporated to dryness in vacuo to afford 2-bromo-5-chloro-4-methylbenzoic acid methyl ester as a whitish solid (26.00 g, 81% yield).

### 5-Bromo-2-chloro-4-(methoxycarbonyl)benzoic acid

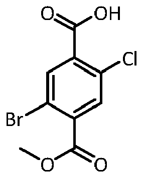

In a round-bottom flask, 2-bromo-5-chloro-4-methylbenzoic acid methyl ester (25.80 g, 97.9 mmol), tetrabutyl ammonium bromide (793 mg, 2.44 mmol), ruthenium chloride trihydrate (200 mg, 0.96 mmol), dichloroethane (26 mL) and sodium hypochlorite 10% (311 mL) were mixed. The pH at the aqueous phase was adjusted to 9.0 by addition of 20% v/v H₂SO₄. The biphasic mixture was stirred for 6 hours and meanwhile NaOH 20% v/v was added to keep the pH at 9. The phases were separated and the aqueous phase was washed with DCM (3 x 50 mL), acidified with 20% H₂SO₄ and extracted with EtOAc (5 x 100 mL). The combined organic fractions were washed with brine (100 mL), dried over anhydrous magnesium sulfate, filtered and evaporated to dryness in vacuo to afford 5-bromo-2-chloro-4-(methoxycarbonyl)benzoic acid as a whitish solid (9.08 g, 32% yield).

### 2-Bromo-5-chloro-4-(4-ethoxybenzoyl)benzoic acid methyl ester [Lv, B. et al. Bioorg Med Chem Lett 2009, 19(24): 6877-81]

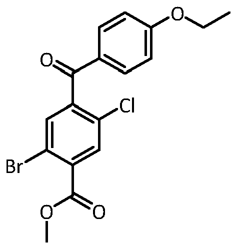

In a round-bottom flask, 5-bromo-2-chloro-4-(methoxycarbonyl)benzoic acid (3.00 g, 10.2 mmol) was dissolved in DCM (90 mL). Thionyl chloride (1.5 mL, 20.4 mmol) and DMF (0.06 mL) were added and the resulting suspension was heated at 50 ºC for 1 hour. The resulting solution was evaporated to dryness in vacuo affording an oily residue, which was dissolved in DCM (90 mL) under inert atmosphere. The resulting solution was cooled to 0 ºC and phenetole (2.0 mL, 15.7 mmol) was added. After cooling to -10 ºC, AlCl₃ (3.05 g, 22.5 mmol) was added portionwise and the resulting suspension was heated at 50 ºC for 1 hour. The mixture was poured into ice/water (200 g), the phases were separated and the aqueous layer extracted with DCM (4 x 50 mL). The combined collected organic layers were washed with brine (3 x 50 mL), dried over anhydrous magnesium sulfate, filtered and evaporated to dryness in vacuo to afford 4.47 g of a residue, which was purified by silica gel column chromatography to afford 2-bromo-5-chloro-4-(4-ethoxybenzoyl)benzoic acid methyl ester as an oil (1.91 g, 47% yield).

### 2-Bromo-5-chloro-4-(4-ethoxybenzyl)benzoic acid methyl ester

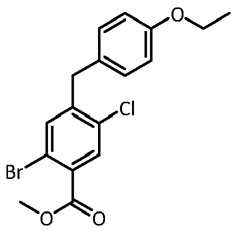

In a round-bottom flask equipped with magnetic stirring and inert atmosphere, 2-bromo-5-chloro-4-(4-ethoxybenzoyl)benzoic acid methyl ester (1.91 g, 4.8 mmol) was dissolved in trifluoroacetic acid (9.5 mL). Firstly, triethylsilane (1.6 mL, 10.1 mmol) was added at room temperature and then over 10 minutes trifluoromethanesulfonic acid (69 µL, 0.76 mmol) was added. The resulting mixture was heated at 75 ºC for 3 hours and then evaporated to dryness in vacuo. The residue was dissolved in EtOAc (150 mL) and washed with water (2 × 50 mL), 10% aqueous sodium hydrogen carbonate (2 × 50 mL) and brine (2 × 50 mL). The organic phase was dried over anhydrous magnesium sulfate, filtered and evaporated to dryness in vacuo to give 3.03 g of a residue, which was purified by silica gel column chromatography to afford 2-bromo-5-chloro-4-(4-ethoxybenzyl)benzoic acid methyl ester (935 mg, 51% yield) as a colorless oil.

### 2-Bromo-5-chloro-4-(4-ethoxybenzyl)benzenemethanol

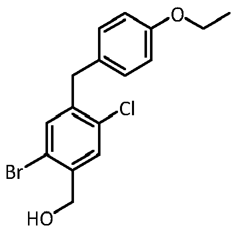

In a round-bottom flask equipped with magnetic stirring and inert atmosphere, 2-bromo-5-chloro-4-(4-ethoxybenzyl)benzoic acid methyl ester (910 mg, 2.37 mmol) was dissolved in anhydrous THF (15 mL). The resulting solution was cooled to 0 ºC with an ice bath and LAH (369 mg, 9.43 mmol) was added portionwise. The mixture was allowed to warm to room temperature and stirred for 2 hours. Water (0.37 mL), 15% aqueous NaOH (0.37 mL) and water (1.11 mL) were added dropwise sequentially. The mixture was stirred for 30 minutes and then filtered through a Celite pad and evaporated to dryness in vacuo to afford 2-bromo-5-chloro-4-(4-ethoxybenzyl)benzenemethanol (820 mg, 97% yield) as a white solid.

### 1-(Allyloxymethyl)-2-bromo-5-chloro-4-(4-ethoxybenzyl)benzene

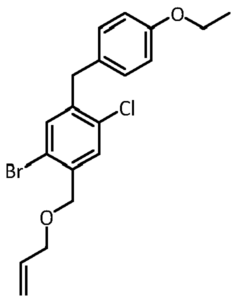

In a round-bottom flask equipped with magnetic stirring and inert atmosphere, 2-bromo-5-chloro-4-(4-ethoxybenzyl)benzenemethanol (815 mg, 2.29 mmol) was dissolved in anhydrous DMF (12 mL). The resulting solution was cooled to 0 ºC with an ice bath and NaH (202 mg, 5.05 mmol) was added portionwise. The mixture was stirred at this temperature for 30 minutes and afterwards tetrabutylammonium iodide (86 mg, 0.23 mmol) and allyl bromide (0.3 mL, 3.27 mmol) were added. The mixture was allowed to warm to room temperature and stirred overnight. Water (5 mL) and EtOAc (20 mL) were added and phases were separated. The aqueous layer was extracted with EtOAc (4 × 10 mL) and the collected combined organic fractions were washed with brine (3 × 20 mL), dried over anhydrous magnesium sulfate, filtered and evaporated to dryness in vacuo to afford 963 mg of a residue which was purified by silica gel column chromatography to afford 1-(allyloxymethyl)-2-bromo-5-chloro-4-(4-ethoxybenzyl)benzene (824 mg, 91% yield) as a colorless oil.

### (2S,3S,4R,5R,6R)-2-[2-(Allyloxymethyl)-4-chloro-5-(4-ethoxybenzyl)phenyl]-3,4,5-tris(benzyloxy)-6-(benzyloxymethyl)tetrahydro-2H-pyran

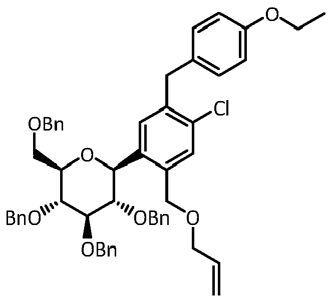

In a round-bottom flask equipped with magnetic stirring and inert atmosphere, 1-(allyloxymethyl)-2-bromo-5-chloro-4-(4-ethoxybenzyl)benzene (800 mg, 2.04 mmol) was dissolved in anhydrous THF (8 mL) and cooled to -78 ºC. *n*-BuLi 1.6 M in hexane (1.4 mL, 2.24 mmol) was added dropwise and the reaction mixture was stirred for 15 minutes at -78 ºC. A freshly prepared solution of (3*R*,4*S*,5*R*,6*R*)-3,4,5-tris(benzyloxy)-6-(benzyloxymethyl)tetrahydro-2*H*-pyran-2-one (1.15 g, 2.13 mmol) in anhydrous THF (4 mL) under inert atmosphere was added dropwise at -78 ºC. The reaction mixture was stirred at this temperature during 1 hour and then allowed to warm to -25 ºC during 2 hours. A saturated ammonium chloride solution was added (10 mL) and the mixture was allowed to warm to room temperature during 30 minutes. Phases were separated and the aqueous layer was extracted with DCM (4 × 25 mL). The combined organic fractions were washed with brine (2 × 25 mL), dried over anhydrous magnesium sulfate, filtered and evaporated to dryness in vacuo to afford 1.86 g of an oily residue. The obtained residue was dissolved in DCM (60 mL) under inert atmosphere and cooled to -25 ºC. Et₃SiH (0.33 mL, 2.06 mmol) and BF₃·Et₂O (0.30 mL, 2.36 mmol) were added sequentially dropwise and then the resulting mixture was allowed to warm to 5 ºC during 3 hours. The mixture was quenched with 10% NaHCO₃ (20 mL) and the phases were separated. The aqueous layer was extracted with DCM (3 × 10 mL) and the combined organic fractions were washed with brine, dried over anhydrous magnesium sulfate, filtered and evaporated to dryness in vacuo to give 1.59 g of an oily residue, which was purified by silica gel column chromatography to afford (2*S*,3*S*,4*R*,5*R*,6*R*)-2-[2-(allyloxymethyl)-4-chloro-5-(4-ethoxybenzyl)phenyl]-3,4,5-tris(benzyloxy)-6-(benzyloxymethyl)tetrahydro-2*H*-pyran (589 mg, 35% yield) as a colorless oil.

### 5-Chloro-4-(4-ethoxybenzyl)-2-[(2S,3S,4R,5R,6R)-3,4,5-tris(benzyloxy)-6-(benzyloxymethyl)tetrahydro-2H-pyran-2-yl]benzenemethanol

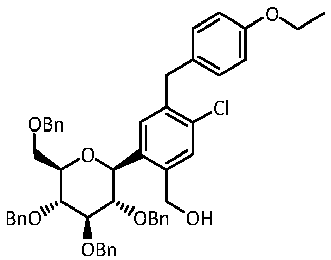

In a round-bottom flask equipped with magnetic stirring, (2*S*,3*S*,4*R*,5*R*,6*R*)-2-[2-(allyloxymethyl)-4-chloro-5-(4-ethoxybenzyl)phenyl]-3,4,5-tris(benzyloxy)-6-(benzyloxymethyl)tetrahydro-2*H*-pyran (580 mg, 0.69 mmol) was dissolved in a 9:1 mixture of acetic acid/water (10 mL) at room temperature. Sodium acetate (225 mg, 2.59 mmol) and PdCl₂ (270 mg, 1.53 mmol) were added and the resulting suspension was heated at 75 ºC for 2 hours. The mixture was then cooled to room temperature, diluted with EtOAc (50 mL) and filtered through a Celite pad. The filtrate was evaporated to dryness in vacuo giving an oily residue, which was purified by silica gel column chromatography to afford 5-chloro-4-(4-ethoxybenzyl)-2-[(2*S*,3*S*,4*R*,5*R*,6*R*)-3,4,5-tris(benzyloxy)-6-(benzyloxymethyl) tetrahydro-2*H*-pyran-2-yl]benzenemethanol (271 mg, 49% yield) as a brownish oil.

### 5-Chloro-4-(4-ethoxybenzyl)-2-[(2S,3S,4R,5R,6R)-3,4,5-tris(benzyloxy)-6-(benzyloxymethyl)tetrahydro-2H-pyran-2-yl]benzoic acid

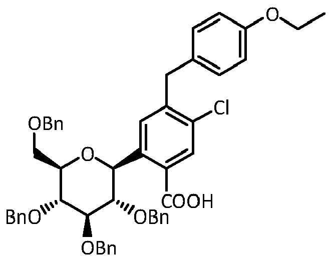

In a round-bottom flask equipped with magnetic stirring, 5-chloro-4-(4-ethoxybenzyl)-2-[(2*S*,3*S*,4*R*,5*R*,6*R*)-3,4,5-tris(benzyloxy)-6-(benzyloxymethyl) tetrahydro-2*H*-pyran-2-yl]benzenemethanol (200 mg, 0.25 mmol) was dissolved in acetone (3 mL) at room temperature and then cooled to 0 ºC with an ice bath. 2.9 M Jones Reagent solution (0.19 mL, 0.54 mmol) was added dropwise and allowed to warm to room temperature. After 2 hours, the blue-green suspension was cooled again and an additional 2.9 M Jones Reagent solution (0.03 mL) was added dropwise and stirred for 4 hours at room temperature. Afterwards, it was diluted with EtOAc (20 mL) and extracted with a saturated aqueous solution of K₂S₂O₃ (3 × 10 mL). The organic fraction was dried over anhydrous magnesium sulfate, filtered and evaporated to dryness in vacuo to afford 5-chloro-4-(4-ethoxybenzyl)-2-[(2*S*,3*S*,4*R*,5*R*,6*R*)-3,4,5-tris(benzyloxy)-6-(benzyloxymethyl)tetrahydro-2*H*-pyran-2-yl]benzoic acid (104 mg, 51% yield) as a colorless oil, which was used in the next step without further purification.

### (2R,3S,4S,4aR,10bS)-8-Chloro-9-(4-ethoxybenzyl)-3,4-dihydroxy-2-(hydroxymethyl)-3,4,4a,10b-tetrahydropyrano[3,2-c][2]benzopyran-6(2H)-one

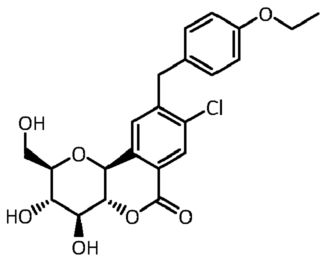

A solution of 5-chloro-4-(4-ethoxybenzyl)-2-[(2*S*,3*S*,4*R*,5*R*,6*R*)-3,4,5-tris(benzyloxy)-6-(benzyloxymethyl)tetrahydro-2*H*-pyran-2-yl]benzoic acid (90 mg, 0.11 mmol) in EtOAc (10 mL) and MeOH (30 mL) was placed in a pressurized reactor. PdCl₂ (15 mg) was added to the solution and the reactor was purged with vacuum and nitrogen. Afterwards, hydrogen was charged up to 3 bar, stirring was continued for 16 hours, and finally, the system was purged again. The suspension was filtered through a Celite pad, washed with methanol and the filtrate was evaporated to dryness under vacuum to give 55 mg of a whitish solid, which was purified by silica gel column chromatography to afford the title compound as a white solid (28 mg, 60% yield).

### Example 3

### (2R,3S,4S,4aR,10bS)-8-Chloro-9-(4-ethylbenzyl)-3,4-dihydroxy-2-(hydroxymethyl)-3,4,4a,10b-tetrahydropyrano[3,2-c][2]benzopyran-6(2H)-one

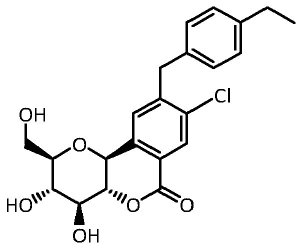

### 2-Bromo-5-chloro-4-(4-ethylbenzoyl)benzoic acid methyl ester [Lv, B. et al. Bioorg Med Chem Lett 2009, 19(24): 6877-81]

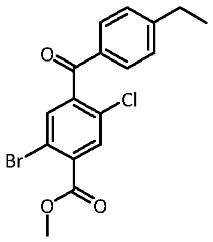

In a round-bottom flask, 5-bromo-2-chloro-4-(methoxycarbonyl)benzoic acid (2.68 g, 9.13 mmol, synthesized as in Example 2) was suspended in DCM (80 mL). Thionyl chloride (1.1 mL, 15.0 mmol) and dimethylformamide (0.05 mL) were added and the resulting suspension was heated at 50 ºC for 1 hour. Afterwards, it was evaporated to dryness in vacuo affording an oily residue, which was dissolved in DCM (70 mL) under inert atmosphere. This solution was cooled to 0 ºC and ethylbenzene (1.3 mL, 10.5 mmol) was added. After cooling to -10 ºC, AlCl₃ (2.72 g, 20.09 mmol) was added portionwise and the resulting suspension was heated at 50 ºC for 1 hour. The mixture was poured into ice/water (70 g), the phases were separated and the aqueous layer extracted with DCM (4 × 75 mL). The combined collected organic layers were washed with brine (3 × 50 mL), dried over anhydrous magnesium sulfate, filtered and evaporated to dryness in vacuo to afford 3.97 g of a residue, which was purified by silica gel column chromatography to afford 2-bromo-5-chloro-4-(4-ethylbenzoyl)benzoic acid methyl ester as an oil (2.08 g, 60% yield).

### 2-Bromo-5-chloro-4-(4-ethylbenzyl)benzoic acid methyl ester

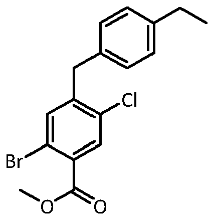

In a round-bottom flask equipped with magnetic stirring and inert atmosphere, 2-bromo-5-chloro-4-(4-ethylbenzoyl)benzoic acid methyl ester (2.08 g, 5.45 mmol) was dissolved in trifluoroacetic acid (11 mL). Firstly, triethylsilane (1.6 mL, 9.91 mmol) was added at room temperature and then over 10 minutes trifluoromethanesulfonic acid (27 µL, 0.30 mmol) was added. The resulting mixture was heated at 75 ºC for 2 hours and then evaporated to dryness in vacuo. The residue was dissolved in EtOAc (100 mL) and washed with water (2 × 50 mL), 10% aqueous sodium hydrogen carbonate (2 × 50 mL) and brine (2 × 50 mL). The organic phase was dried over anhydrous magnesium sulfate, filtered and evaporated to dryness in vacuo to give 2.69 g of a residue, which was purified by silica gel column chromatography to afford 2-bromo-5-chloro-4-(4-ethylbenzyl)benzoic acid methyl ester (1.69 g, 85% yield) as a yellowish oil.

### 2-Bromo-5-chloro-4-(4-ethylbenzyl)benzenemethanol

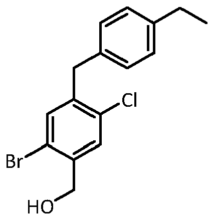

In a round-bottom flask equipped with magnetic stirring and inert atmosphere, 2-bromo-5-chloro-4-(4-ethylbenzyl)benzoic acid methyl ester (2.41 g, 6.55 mmol) was dissolved in anhydrous THF (40 mL). The resulting solution was cooled to 0 ºC with an ice bath and LAH (1.02 g, 26.2 mmol) was added portionwise. The mixture was allowed to warm to room temperature and stirred for 3 hours. Water (1 mL), 15% aqueous NaOH (1 mL) and water (3 mL) were added dropwise sequentially. The mixture was stirred for 30 minutes and then filtered through a Celite pad and evaporated to dryness in vacuo to afford 2-bromo-5-chloro-4-(4-ethylbenzyl) benzenemethanol (2.23 g, 100% yield) as a white waxy solid.

### 1-(Allyloxymethyl)-2-bromo-5-chloro-4-(4-ethylbenzyl)benzene

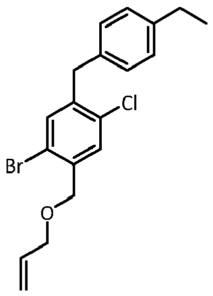

In a round-bottom flask equipped with magnetic stirring and inert atmosphere, 2-bromo-5-chloro-4-(4-ethylbenzyl)benzenemethanol (2.23 g, 6.57 mmol) was dissolved in anhydrous DMF (33 mL). The resulting solution was cooled to 0 ºC with an ice bath and NaH (578 mg, 14.4 mmol) was added portionwise. The mixture was stirred at this temperature for 30 minutes and afterwards tetrabutylammonium iodide (245 mg, 0.66 mmol) and allyl bromide (0.71 mL, 7.88 mmol) were added. The mixture was allowed to warm to room temperature and stirred overnight. Water (15 mL) and EtOAc (50 mL) were added and phases were separated. The aqueous layer was extracted with EtOAc (4 × 50 mL) and the collected combined organic fractions were washed with brine (3 × 50 mL), dried over anhydrous magnesium sulfate, filtered and evaporated to dryness in vacuo to afford 3.04 g of a residue, which was purified by silica gel column chromatography to afford 1-(allyloxymethyl)-2-bromo-5-chloro-4-(4-ethylbenzyl)benzene (2.24 g, 90% yield) as a colorless oil.

### (2S,3S,4R,5R,6R)-2-[2-(Allyloxymethyl)-4-chloro-5-(4-ethylbenzyl)phenyl]-3,4,5-tris(benzyloxy)-6-(benzyloxymethyl)tetrahydro-2H-pyran

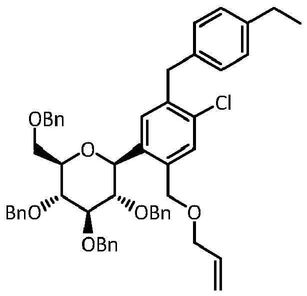

In a round-bottom flask equipped with magnetic stirring and inert atmosphere, 1-(allyloxymethyl)-2-bromo-5-chloro-4-(4-ethylbenzyl)benzene (2.06 g, 5.42 mmol) was dissolved in anhydrous THF (20 mL) and cooled to -78 ºC. n-BuLi 1.6 M in hexane (3.5 mL, 5.6 mmol) was added dropwise and the reaction mixture was stirred for 15 minutes at -78 ºC. A freshly prepared solution of (3*R*,4*S*,5*R*,6*R*)-3,4,5-tris(benzyloxy)-6-(benzyloxymethyl)tetrahydro-2*H*-pyran-2-one (2.97 g, 5.51 mmol) in anhydrous THF (10 mL) under inert atmosphere was added dropwise at -78 ºC. The reaction mixture was stirred at this temperature during 1 hour and then allowed to warm to -45 ºC during 2 hours. A saturated ammonium chloride solution was added (26 mL) and the mixture was allowed to warm to room temperature during 30 minutes. Phases were separated and the aqueous layer was extracted with DCM (4 × 50 mL). The combined organic fractions were washed with brine (2 × 50 mL), dried over anhydrous magnesium sulfate, filtered and evaporated to dryness in vacuo to afford 4.98 g of an oily residue. The obtained residue was dissolved in DCM (160 mL) under inert atmosphere and cooled to -25 ºC. Et₃SiH (0.86 mL, 5.38 mmol) and BF₃·Et₂O (0.79 mL, 6.25 mmol) were added sequentially dropwise and then the resulting mixture was allowed to warm to 5 ºC during 2 hours. The mixture was quenched with 10% NaHCO₃ (50 mL) and the phases were separated. The aqueous layer was extracted with DCM (3 x 25 mL) and the combined organic fractions were washed with brine, dried over anhydrous magnesium sulfate, filtered and evaporated to dryness in vacuo to give 4.63 g of an oily residue, which was purified by silica gel column chromatography to afford (2*S*,3*S*,4*R*,5*R*,6*R*)-2-[2-(allyloxymethyl)-4-chloro-5-(4-ethylbenzyl)phenyl]-3,4,5-tris(benzyloxy)-6-(benzyloxymethyl)tetrahydro-2*H*-pyran (1.25 g, 28% yield) as a colorless oil.

### 5-Chloro-4-(4-ethylbenzyl)-2-[(2S,3S,4R,5R,6R)-3,4,5-tris(benzyloxy)-6-(benzyloxymethyl)tetrahydro-2H-pyran-2-yl]benzenemethanol

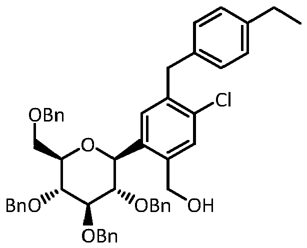

In a round-bottom flask equipped with magnetic stirring, (2*S*,3*S*,4*R*,5*R*,6*R*)-2-[2-(allyloxymethyl)-4-chloro-5-(4-ethylbenzyl)phenyl]-3,4,5-tris(benzyloxy)-6-(benzyloxymethyl)tetrahydro-2*H*-pyran (1.20 g, 1.46 mmol) was dissolved in a 9:1 mixture of acetic acid/water (20 mL) at room temperature. Sodium acetate (476 mg, 5.48 mmol) and PdCl₂ (566 mg, 3.21 mmol) were added and the resulting suspension was heated at 75 ºC for 1.5 hours. Then, the mixture was cooled to room temperature, diluted with EtOAc (100 mL) and filtered through a Celite pad. The filtrate was evaporated to dryness in vacuo giving an oily residue, which was purified by silica gel column chromatography to afford 5-chloro-4-(4-ethylbenzyl)-2-[(2*S*,3*S*,4*R*,5*R*,6*R*)-3,4,5-tris(benzyloxy)-6-(benzyloxymethyl) tetrahydro-2*H*-pyran-2-yl]benzenemethanol (701 mg, 61% yield) as a brownish oil.

### 5-Chloro-4-(4-ethylbenzyl)-2-[(2S,3S,4R,5R,6R)-3,4,5-tris(benzyloxy)-6-(benzyloxymethyl)tetrahydro-2H-pyran-2-yl]benzoic acid

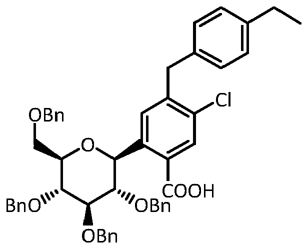

In a round-bottom flask equipped with magnetic stirring, 5-chloro-4-(4-ethylbenzyl)-2-[(2*S*,3*S*,4*R*,5*R*,6*R*)-3,4,5-tris(benzyloxy)-6-(benzyloxymethyl)tetrahydro-2*H*-pyran-2-yl]benzenemethanol (690 mg, 0.88 mmol) was dissolved in acetone (10 mL) at room temperature and then cooled to 0 ºC with an ice bath. 2.9 M Jones Reagent solution (0.60 mL, 1.74 mmol) was added dropwise and allowed to warm to room temperature. After 2 hours, the blue-green suspension was cooled again and an additional 2.9 M Jones Reagent solution (0.1 mL) was added dropwise and stirred for 4 hours at room temperature. Afterwards, it was diluted with EtOAc (70 mL) and extracted with a saturated aqueous solution of K₂S₂O₃ (3 × 30 mL). The organic fraction was dried over anhydrous magnesium sulfate, filtered and evaporated to dryness in vacuo to afford 5-chloro-4-(4-ethylbenzyl)-2-[(2*S*,3*S*,4*R*,5*R*,6*R*)-3,4,5-tris(benzyloxy)-6-(benzyloxymethyl)tetrahydro-2*H*-pyran-2-yl]benzoic acid (678 mg, 96% yield) as a colorless oil, which was used in the next step without further purification.

### (2R,3S,4S,4aR,10bS)-8-Chloro-9-(4-ethylbenzyl)-3,4-dihydroxy-2-(hydroxymethyl)-3,4,4a,10b-tetrahydropyrano[3,2-c][2]benzopyran-6(2H)-one

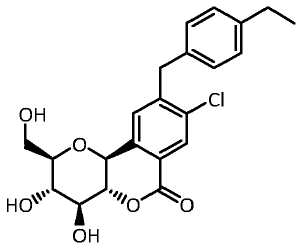

A solution of 5-chloro-4-(4-ethylbenzyl)-2-[(2*S*,3*S*,4*R*,5*R*,6*R*)-3,4,5-tris(benzyloxy)-6-(benzyloxymethyl)tetrahydro-2*H*-pyran-2-yl]benzoic acid (670 mg, 0.84 mmol) in EtOAc (20 mL) and MeOH (50 mL) was placed in a pressurized reactor. PdCl₂ (94 mg) was added to the solution and the reactor was purged with vacuum and nitrogen. Afterwards, hydrogen was charged up to 3 bar, stirring was continued for 16 hours, and finally, the system was purged again. The suspension was filtered through a Celite pad, washed with methanol and the filtrate was evaporated to dryness under vacuum to give 430 mg of a whitish solid, which was purified by silica gel column chromatography to afford the title compound as a white solid (257 mg, 73% yield).

### Example 4

### (2R,3S,4S,4aR,10bS)-9-(4-Ethoxybenzyl)-3,4-dihydroxy-2-(hydroxymethyl)-3,4,4a,10b-tetrahydropyrano[3,2-c][2]benzopyran-6(2H)-one

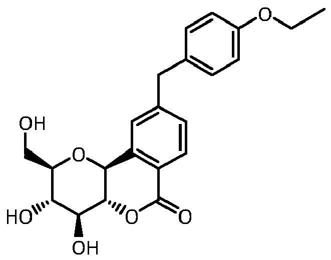

### 2-Bromo-4-(4-ethoxybenzoyl)benzoic acid methyl ester

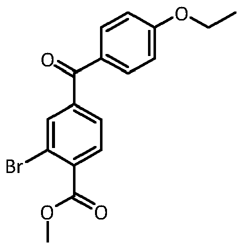

In a round-bottom flask, 3-bromo-4-methoxycarbonylbenzoic acid (5.00 g, 19.3 mmol, synthesized as in Example 1) was dissolved in DCM (150 mL). Thionyl chloride (2.3 mL, 31.3 mmol) and DMF (0.1 mL) were added and the resulting suspension was heated at 50 ºC for 1 hour. The resulting solution was evaporated to dryness *in vacuo* affording an oily residue, which was dissolved in DCM (150 mL) and cooled to 0 ºC under inert atmosphere. Phenetole (2.7 mL, 21.0 mmol) was added and the resulting mixture was cooled to -10 ºC. After cooling, AlCl₃ (5.76 g, 42.5 mmol) was added portionwise. The resulting suspension was heated at 50 ºC for 1.5 hours and then poured into ice/water (400 g). The phases were separated and the aqueous layer extracted with DCM (4 × 75 mL). The combined organic fractions were washed with brine (100 mL), dried over anhydrous magnesium sulfate, filtered and evaporated to dryness in vacuo to afford 6.26 g of a crude product, which was purified by silica gel column chromatography to afford 2-bromo-4-(4-ethoxybenzoyl)benzoic acid methyl ester as an oil (4.35 g, 62% of yield).

### 2-Bromo-4-(4-ethoxybenzyl)benzoic acid methyl ester

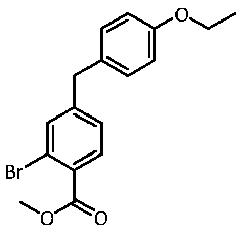

In a round-bottom flask equipped with magnetic stirring and inert atmosphere, 2-bromo-4-(4-ethoxybenzoyl)benzoic acid methyl ester (4.20 g, 11.6 mmol) was dissolved in trifluoroacetic acid (20 mL). Firstly, triethylsilane (3.8 mL, 23.6 mmol) was added at room temperature and then over 10 minutes trifluoromethanesulfonic acid (166 µL, 1.85 mmol) was added. The resulting mixture was heated at 75 ºC for 2.5 hours and then evaporated to dryness *in vacuo.* The residue was dissolved in EtOAc (400 mL) and washed with water (3 × 100 mL), 10% aqueous sodium hydrogen carbonate (3 x 100 mL) and brine (3 × 100 mL). The organic phase was dried over anhydrous magnesium sulfate, filtered and evaporated to dryness in vacuo to give 6.87 g of a residue, which was purified by silica gel column chromatography to afford 2-bromo-4-(4-ethoxybenzyl)benzoic acid methyl ester (2.34 g, 58% yield) as a colorless oil.

### 2-Bromo-4-(4-ethoxybenzyl)benzenemethanol

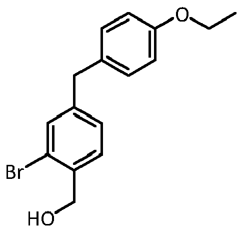

In a round-bottom flask equipped with magnetic stirring and inert atmosphere, 2-bromo-4-(4-ethoxybenzyl)benzoic acid methyl ester (2.2 g, 6.30 mmol) was dissolved in anhydrous THF (40 mL). The resulting solution was cooled to 0 ºC with an ice bath and LAH (0.99 g, 25.2 mmol) was added portionwise. The mixture was allowed to warm to room temperature and stirred for 3 hours. Water (1.0 mL), 15% aqueous NaOH (1.0 mL) and water (3.0 mL) were added dropwise sequentially. The mixture was stirred for 30 minutes and then filtered through a Celite pad and evaporated to dryness in vacuo to afford 2-bromo-4-(4-ethoxybenzyl) benzenemethanol (1.96 g, 97% yield) as a white waxy solid.

### 1-(Allyloxymethyl)-2-bromo-4-(4-ethoxybenzyl)benzene

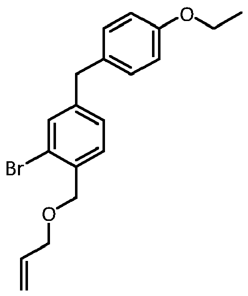

In a round-bottom flask equipped with magnetic stirring and inert atmosphere, 2-bromo-4-(4-ethoxybenzyl)benzenemethanol (1.90 g, 5.91 mmol) was dissolved in anhydrous DMF (25 mL). The resulting solution was cooled to 0 ºC with an ice bath and NaH (473 mg, 11.82 mmol) was added portionwise. The mixture was stirred at this temperature for 30 minutes and afterwards tetrabutylammonium iodide (224 mg, 0.6 mmol) and allyl bromide (0.65 mL, 7.09 mmol) were added. The mixture was allowed to warm to room temperature and stirred overnight. Water (10 mL) and EtOAc (50 mL) were added and phases were separated. The aqueous layer was extracted with EtOAc (3 x 50 mL) and the collected combined organic fractions were washed with brine (5 × 50 mL), dried over anhydrous magnesium sulfate, filtered and evaporated to dryness in vacuo to afford 2.45 g of a residue, which was purified by silica gel column chromatography to afford 1-(allyloxymethyl)-2-bromo-4-(4-ethoxybenzyl)benzene (2.01 g, 94% yield) as a colorless oil.

### (2S,3S,4R,5R,6R)-2-[2-(Allyloxymethyl)-5-(4-ethoxybenzyl)phenyl]-3,4,5-tris(benzyloxy)-6-(benzyloxymethyl)tetrahydro-2H-pyran

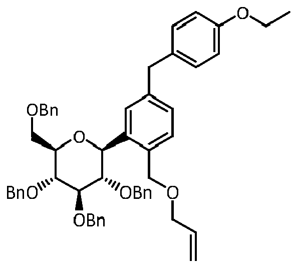

In a round-bottom flask equipped with magnetic stirring and inert atmosphere, 1-(allyloxymethyl)-2-bromo-4-(4-ethoxybenzyl)benzene (1.90 g, 5.26 mmol) was dissolved in anhydrous THF (30 mL) and cooled to -78 ºC. n-BuLi 1.6 M in hexanes (3.6 mL, 5.76 mmol) was added dropwise and the reaction mixture was stirred for 20 minutes at -78 ºC. A freshly prepared solution of (3*R*,4*S*,5*R*,6*R*)-3,4,5-tris(benzyloxy)-6-(benzyloxymethyl)tetrahydro-2*H*-pyran-2-one (3.12 g, 5.79 mmol) in anhydrous THF (10 mL) under inert atmosphere was added dropwise at -78 ºC. The reaction mixture was stirred and allowed to warm to -5 ºC during 3 hours. A saturated ammonium chloride solution was added (25 mL) and the mixture was allowed to warm to room temperature during 1 hour. Phases were separated and the aqueous layer was extracted with DCM (4 × 50 mL). The combined organic fractions were washed with brine (3 x 50 mL), dried over anhydrous magnesium sulfate, filtered and evaporated to dryness in vacuo to afford 4.48 g of an oily residue. The obtained product was dissolved in DCM (150 mL) under inert atmosphere and cooled to -25 ºC. Et₃SiH (0.84 mL, 5.26 mmol) and BF₃·Et₂O (0.70 mL, 5.51 mmol) were added sequentially dropwise and then the resulting mixture was allowed to warm to 5 ºC during 2 hours. The mixture was quenched with 10% NaHCO₃ (50 mL) and the phases were separated. The aqueous layer was extracted with DCM (3 × 50 mL) and the combined organic fractions were washed with brine, dried over anhydrous magnesium sulfate, filtered and evaporated to dryness in vacuo to give 4.32 g of an oily residue, which was purified by silica gel column chromatography to afford (2*S*,3*S*,4*R*,5*R*,6*R*)-2-[2-(allyloxymethyl)-5-(4-ethoxybenzyl)phenyl]-3,4,5-tris(benzyloxy)-6-(benzyloxymethyl)tetrahydro-2*H*-pyran (804 mg, 19% yield) as a colorless oil.

### 4-(4-Ethoxybenzyl)-2-[(2S,3S,4R,5R,6R)-3,4,5-tris(benzyloxy)-6-(benzyloxymethyl) tetrahydro-2H-pyran-2-yl]benzenemethanol

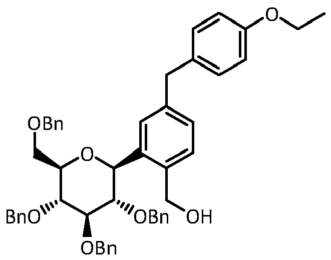

In a round-bottom flask equipped with magnetic stirring, (2*S*,3*S*,4*R*,5*R*,6*R*)-2-[2-(allyloxymethyl)-5-(4-ethoxybenzyl)phenyl]-3,4,5-tris(benzyloxy)-6-(benzyloxymethyl)tetrahydro-2*H*-pyran (790 mg, 0.98 mmol) was dissolved in a 9:1 mixture of acetic acid/water (10 mL) at room temperature. Sodium acetate (317 mg, 3.65 mmol) and PdCl₂ (377mg, 2.13 mmol) were added and the resulting suspension was heated at 70 ºC for 80 minutes. Afterwards, it was cooled to room temperature, diluted with EtOAc and filtered through a Celite pad. The filtrate was evaporated to dryness in vacuo giving an oily residue, which was purified by silica gel column chromatography to afford 4-(4-ethoxybenzyl)-2-[(2*S,*3*S*,4*R*,5*R*,6*R*)-3,4,5-tris(benzyloxy)-6-(benzyloxymethyl)tetrahydro-2*H*-pyran-2-yl]benzene methanol (405 mg, 54% yield) as a colorless oil.

### 4-(4-Ethoxybenzyl)-2-[(2S,3S,4R,5R,6R)-3,4,5-tris(benzyloxy)-6-(benzyloxymethyl) tetrahydro-2H-pyran-2-yl]benzoic acid

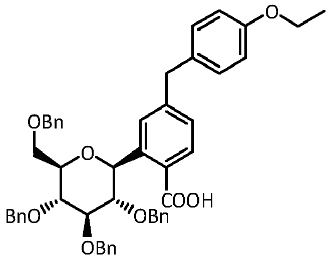

In a round-bottom flask equipped with magnetic stirring, 4-(4-ethoxybenzyl)-2-[(2*S*,3*S*,4*R*,5*R*,6*R*)-3,4,5-tris(benzyloxy)-6-(benzyloxymethyl)tetrahydro-2*H*-pyran-2-yl]benzenemethanol (400 mg, 0.52 mmol) was dissolved in acetone (6 mL) at room temperature and then cooled to 0 ºC with an ice bath. 2.9 M Jones Reagent solution (0.35 mL, 1.04 mmol) was added dropwise and allowed to warm to room temperature. After 2 hours, the blue-green suspension was cooled again and an additional 2.9 M Jones Reagent solution (0.06 mL) was added dropwise and stirred for 4 hours at room temperature. Afterwards, it was diluted with EtOAc (50 mL) and extracted with a saturated aqueous solution of K₂S₂O₃ (3 × 15 mL). The organic fraction was dried over anhydrous magnesium sulfate, filtered and evaporated to dryness in vacuo to afford 4-(4-ethoxybenzyl)-2-[(2*S*,3*S*,4*R*,5*R*,6*R*)-3,4,5-tris(benzyloxy)-6-(benzyloxymethyl)tetrahydro-2*H*-pyran-2-yl]benzoic acid (344 mg, 85% yield) as a colorless oil, which was used in the next step without further purification.

### (2R,3S,4S,4aR,10bS)-9-(4-Ethoxybenzyl)-3,4-dihydroxy-2-(hydroxymethyl)-3,4,4a,10b-tetrahydropyrano[3,2-c][2]benzopyran-6(2H)-one

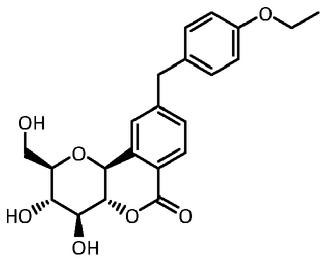

A solution of 4-(4-ethoxybenzyl)-2-[(2*S*,3*S*,4*R*,5*R*,6*R*)-3,4,5-tris(benzyloxy)-6-(benzyloxymethyl)tetrahydro-2*H*-pyran-2-yl]benzoic acid (315 mg, 0.40 mmol) in EtOAc (10 mL) and MeOH (50 mL) was placed in a pressurized reactor. PdCl₂ (45 mg) was added to the solution and the reactor was purged with vacuum and nitrogen. Afterwards, hydrogen was charged up to 3 bar, stirring was continued for 16 hours, and finally, the system was purged again. The suspension was filtered through a Celite pad and washed with methanol. The filtrate was evaporated to dryness under vacuum to give 350 mg of a whitish solid, which was purified by silica gel column chromatography to afford the title compound as a whitish solid (93 mg, 58% yield).

### Example 5

### (2R,3S,4S,4aR,10bS)-9-(4-Ethylbenzyl)-3,4-dihydroxy-2-(ethoxycarbonyloxymethyl)-3,4,4a,10b-tetrahydropyrano[3,2-c][2]benzopyran-6(2H)-one

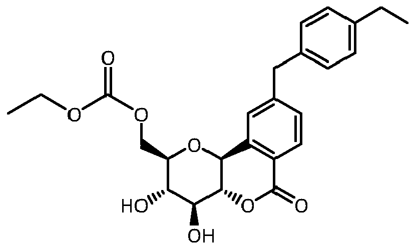

In a round-bottom flask equipped with magnetic stirring and inert atmosphere, (2*R*,3*S*,4*S*,4a*R*,10b*S*)-9-(4-ethylbenzyl)-3,4-dihydroxy-2-(hydroxymethyl)-3,4,4a,10b-tetrahydropyrano[3,2-*c*][2]benzopyran-6(2*H*)-one (100 mg, 0.26 mmol, Example 1) was dissolved in 2,4,6-trimethylpyridine (1.5 mL). The resulting solution was cooled to 0 ºC with an ice bath and ethyl chloroacetylformiate (0.7 mL, 7.2 mmol) was added dropwise. The mixture was allowed to warm to room temperature and stirred overnight. The reaction mixture was poured into aqueous 10% citric acid (15 mL) and extracted with EtOAc (3 × 30 mL). The collected combined organic fractions were washed with brine (2 × 30 mL), dried over anhydrous magnesium sulfate, filtered and evaporated to dryness in vacuo to afford 135 mg of a residue, which was purified by silica gel column chromatography to afford the title compound (89 mg, 75% yield) as a white solid.

### Example 6

### (2R,3S,4S,4aR,10bS)-3,4-Dihydroxy-2-(hydroxymethyl)-9-(4-methoxybenzyl)-3,4,4a,10b-tetrahydropyrano[3,2-c][2]benzopyran-6(2H)-one

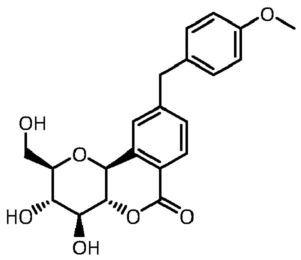

### 2-Bromo-4-(4-methoxybenzoyl)benzoic acid methyl ester

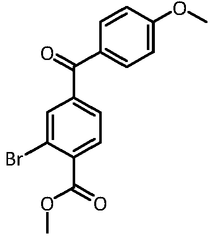

In a round-bottom flask, 3-bromo-4-methoxycarbonylbenzoic acid (15.03 g, 57.9 mmol, synthesized as in Example 1) was dissolved in DCM (300 mL). Thionyl chloride (7 mL, 95.2 mmol) and DMF (0.3 mL) were added and the resulting suspension was heated at 50 ºC for 1 hour. The resulting solution was evaporated to dryness in vacuo affording an oily residue, which was dissolved in DCM (300 mL) under inert atmosphere and anisol (7 mL, 63.7 mmol) was added. After cooling to 0 ºC with an ice bath, AlCl₃ (19.6 g, 144.8 mmol) was added portionwise. The resulting suspension was stirred at room temperature for 6 hours and then poured into ice/water (600 g). The phases were separated and the aqueous layer extracted with DCM (4 × 50 mL). The combined organic fractions were washed with aqueous 10% HCl (4 × 50 mL), aqueous 10% NaOH (4 × 50 mL) and brine (100 mL), dried over anhydrous magnesium sulfate, filtered and evaporated to dryness in vacuo to afford 16.72 g of a crude product, which was purified by silica gel column chromatography to afford 2-bromo-4-(4-methoxybenzoyl)benzoic acid methyl ester as an oil (9.92 g, 49% yield).

### 2-Bromo-4-(4-methoxybenzyl)benzoic acid methyl ester

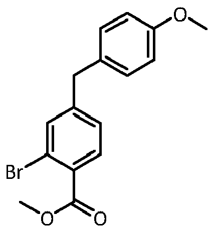

In a round-bottom flask equipped with magnetic stirring and inert atmosphere, 2-bromo-4-(4-methoxybenzoyl)benzoic acid methyl ester (9.00 g, 25.77 mmol) was dissolved in trifluoroacetic acid (50 mL). Firstly, triethylsilane (8.30 mL, 51.47 mmol) was added at room temperature and then over 10 minutes trifluoromethanesulfonic acid (73 µL, 0.81 mmol) was added. The resulting mixture was heated at 75 ºC for 3 hours and then evaporated to dryness in vacuo. The residue was dissolved in EtOAc (300 mL) and washed with water (3 × 100 mL), 10% aqueous sodium hydrogen carbonate (4 × 100 mL) and brine (3 × 100 mL). The organic phase was dried over anhydrous magnesium sulfate, filtered and evaporated to dryness in vacuo to give 11.23 g of a residue, which was purified by silica gel column chromatography to afford 2-bromo-4-(4-methoxybenzyl)benzoic acid methyl ester (5.01 g, 58% yield) as a colorless oil.

### 2-Bromo-4-(4-methoxybenzyl)benzenemethanol

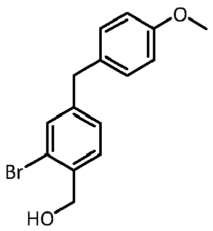

In a round-bottom flask equipped with magnetic stirring and inert atmosphere, 2-bromo-4-(4-methoxybenzyl)benzoic acid methyl ester (4.5 g, 13.43 mmol) was dissolved in anhydrous THF (80 mL). The resulting solution was cooled to 0 ºC with an ice bath and LAH (2.10 g, 53.72 mmol) was added portionwise. The mixture was allowed to warm to room temperature and stirred for 2.5 hours. Water (2.1 mL), 15% aqueous NaOH (2.1 mL) and water (6.3 mL) were added dropwise sequentially. The mixture was stirred for 45 minutes and then filtered through a Celite pad and evaporated to dryness in vacuo to afford 2-bromo-4-(4-methoxybenzyl)benzene methanol (3.96 g, 96% yield) as a waxy white solid.

### 1-(Allyloxymethyl)-2-bromo-4-(4-methoxybenzyl)benzene

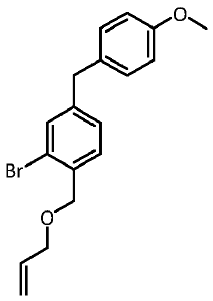

In a round-bottom flask equipped with magnetic stirring and inert atmosphere, 2-bromo-4-(4-methoxybenzyl)benzenemethanol (3.80 g, 12.4 mmol) was dissolved in anhydrous DMF (60 mL). The resulting solution was cooled to 0 ºC with an ice bath and NaH (1.09 g, 27.28 mmol) was added portionwise. The mixture was stirred at this temperature for 30 minutes and afterwards tetrabutylammonium iodide (464 mg, 1.24 mmol) and allyl bromide (1.4 mL, 15.27 mmol) were added. The mixture was allowed to warm to room temperature and stirred overnight. Water (30 mL) and EtOAc (100 mL) were added and phases were separated. The aqueous layer was extracted with EtOAc (3 × 50 mL) and the collected combined organic fractions were washed with brine (5 × 75 mL), dried over anhydrous magnesium sulfate, filtered and evaporated to dryness in vacuo to afford 5.04 g of a residue, which was purified by silica gel column chromatography to afford 1-(allyloxymethyl)-2-bromo-4-(4-methoxybenzyl)benzene (3.87 g, 90% yield) as a colorless oil.

### (2S,3S,4R,5R,6R)-2-[2-(Allyloxymethyl)-5-(4-methoxybenzyl)phenyl]-3,4,5-tris(benzyloxy)-6-(benzyloxymethyl)tetrahydro-2H-pyran

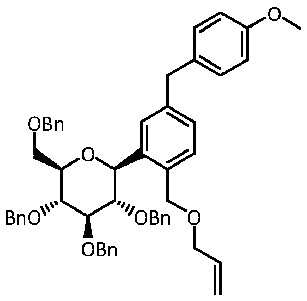

In a round-bottom flask equipped with magnetic stirring and inert atmosphere, 1-(allyloxymethyl)-2-bromo-4-(4-methoxybenzyl)benzene (3.50 g, 10.08 mmol) was dissolved in anhydrous THF (60 mL) and cooled to -78 ºC. *n*-BuLi 1.6 M in hexane (6.5 mL, 10.4 mmol) was added dropwise and the reaction mixture was stirred for 30 minutes at -78 ºC. A freshly prepared solution of (3*R*,4*S*,5*R*,6*R*)-3,4,5-tris(benzyloxy)-6-(benzyloxymethyl)tetrahydro-2*H*-pyran-2-one (5.51 g, 10.21 mmol) in anhydrous THF (20 mL) under inert atmosphere was added dropwise at -78 ºC. The reaction mixture was stirred at this temperature during 1 hour and then allowed to warm to -5 ºC during 3 hours. A saturated ammonium chloride solution was added (50 mL) and the mixture was allowed to warm to room temperature during 45 minutes. Phases were separated and the aqueous layer was extracted with DCM (4 × 100 mL). The combined organic fractions were washed with brine (3 x 100 mL), dried over anhydrous magnesium sulfate, filtered and evaporated to dryness in vacuo to afford 9.81 g of an oily residue. The obtained residue was dissolved in DCM (250 mL) under inert atmosphere and cooled to -25 ºC. Et₃SiH (1.59 mL, 9.95 mmol) and BF₃·Et₂O (1.4 mL, 10.8 mmol) were added sequentially dropwise and the resulting mixture was allowed to warm to 5 ºC during 3 hours. The mixture was quenched with 10% NaHCO₃ (100 mL) and the phases were separated. The aqueous layer was extracted with DCM (3 x 50 mL) and the combined organic fractions were washed with brine, dried over anhydrous magnesium sulfate, filtered and evaporated to dryness in vacuo to give 7.63 g of an oily residue, which was purified by silica gel column chromatography to afford (2*S*,3*S*,4*R*,5*R*,6*R*)-2-[2-(allyloxymethyl)-5-(4-methoxybenzyl)phenyl]-3,4,5-tris(benzyloxy)-6-(benzyloxymethyl)tetrahydro-2*H*-pyran (1.99 g, 25% yield) as a colorless oil.

### 4-(4-Methoxybenzyl)-2-[(2S,3S,4R,5R,6R)-3,4,5-tris(benzyloxy)-6-(benzyloxymethyl)tetrahydro-2H-pyran-2-yl]benzenemethanol

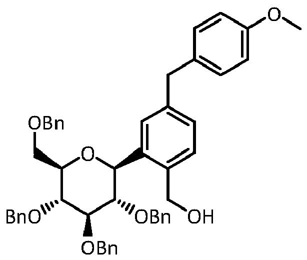

In a round-bottom flask equipped with magnetic stirring (2*S*,3*S*,4*R*,5*R*,6*R*)-2-[2-(allyloxymethyl)-5-(4-methoxybenzyl)phenyl]-3,4,5-tris(benzyloxy)-6-(benzyloxymethyl)tetrahydro-2*H*-pyran (1.80 g, 2.28 mmol) was dissolved in a 9:1 mixture of acetic acid/water (20 mL) at room temperature. Sodium acetate (737 mg, 8.48 mmol) and PdCl₂ (876 mg, 4.95 mmol) were added and the resulting suspension was heated at 70 ºC for 90 minutes. Then, the mixture was cooled to room temperature, diluted with EtOAc and filtered through a Celite pad. The filtrate was evaporated to dryness in vacuo giving an oily residue, which was purified by silica gel column chromatography to afford 4-(4-methoxybenzyl)-2-[(2*S*,3*S*,4*R*,5*R*,6*R*)-3,4,5-tris(benzyloxy)-6-(benzyloxymethyl)tetrahydro-2*H*-pyran-2-yl]benzenemethanol (650 mg, 38% yield) as a colorless oil.

### 4-(4-Methoxybenzyl)-2-[(2S,3S,4R,5R,6R)-3,4,5-tris(benzyloxy)-6-(benzyloxymethyl)tetrahydro-2H-pyran-2-yl]benzoic acid

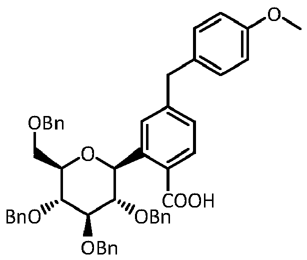

In a round-bottom flask equipped with magnetic stirring, 4-(4-methoxybenzyl)-2-[(2*S*,3*S*,4*R*,5*R*,6*R*)-3,4,5-tris(benzyloxy)-6-(benzyloxymethyl)tetrahydro-2*H*-pyran-2-yl]benzenemethanol (630 mg, 0.83 mmol) was dissolved in acetone (9 mL) at room temperature and then cooled to 0 ºC with an ice bath. 2.9 M Jones Reagent solution (0.57 mL, 1.67 mmol) was added dropwise and allowed to warm to room temperature. After 2 hours, the blue-green suspension was cooled again and an additional 2.9 M Jones Reagent solution (0.10 mL) was added dropwise and stirred for 4 hours at room temperature. Afterwards, it was diluted with EtOAc (75 mL) and extracted with a saturated aqueous solution of K₂S₂O₃ (3 × 20 mL). The organic fraction was dried over anhydrous magnesium sulfate, filtered and evaporated to dryness in vacuo to afford 4-(4-methoxybenzyl)-2-[(2*S*,3*S*,4*R*,5*R*,6*R*)-3,4,5-tris(benzyloxy)-6-(benzyloxymethyl)tetrahydro-2*H*-pyran-2-yl]benzoic acid (564 mg, 88% yield) as a brownish oil, which was used in next step without further purification.

### (2R,3S,4S,4aR,10bS)-3,4-Dihydroxy-2-(hydroxymethyl)-9-(4-methoxybenzyl)-3,4,4a,10b-tetrahydropyrano[3,2-c][2]benzopyran-6(2H)-one

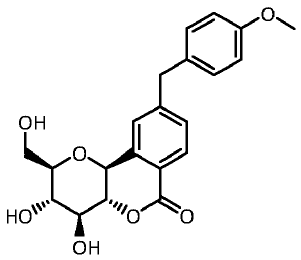

A solution of 4-(4-methoxybenzyl)-2-[(2*S*,3*S*,4*R*,5*R*,6*R*)-3,4,5-tris(benzyloxy)-6-(benzyloxymethyl)tetrahydro-2*H*-pyran-2-yl]benzoic acid (500 mg, 0.65 mmol) in EtOAc (20 mL) and MeOH (60 mL) was placed in a pressurized reactor. PdCl₂ (74 mg) was added to the solution and the reactor was purged with vacuum and nitrogen. Afterwards, hydrogen was charged up to 3 bar, stirring was continued for 16 hours, and finally, the system was purged again. The suspension was filtered through a Celite pad, washed with methanol and the filtrate was evaporated to dryness under vacuum to afford 286 mg of a whitish solid, which was purified by silica gel column chromatography to afford the title compound as a whitish solid (151 mg, 60% yield).

The compounds of Examples 7 to 12 are presented in Table 1. These compounds are prepared employing the procedures of reaction schemes 1 to 8 above. The starting materials for these compounds are commercially available.

**Table 1**

| | | | | |
|---|---|---|---|---|
| 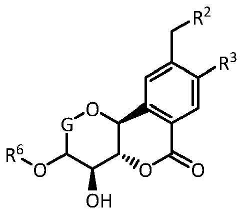 | | | | |

| **Example** | **G** | **OR⁶** | **R²** | **R³** |
|---|---|---|---|---|
| 7 | 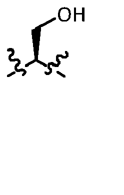 | 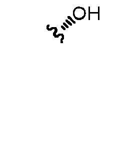 | 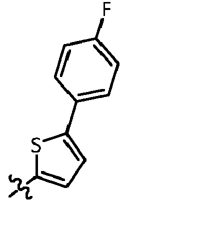 | Me |
| 8 | 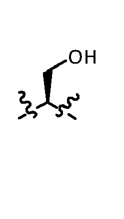 | 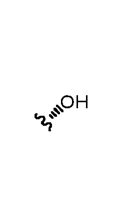 | 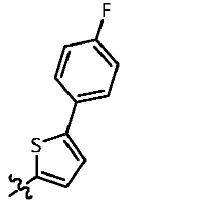 | Cl |
| 9 | 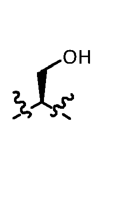 | 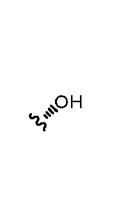 | 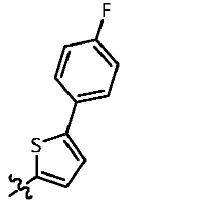 | H |
| 10 | 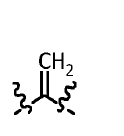 | 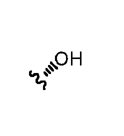 | 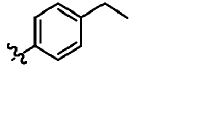 | H |
| 11 | 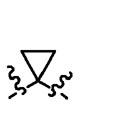 | 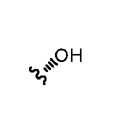 | 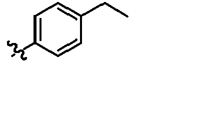 | H |
| 12 | 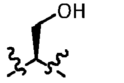 | 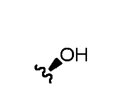 | 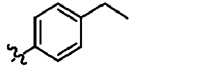 | H |

### BIOLOGICAL ASSAYS

The following examples illustrate the biological activity of compounds of formula (I) and should not be considered as limiting the scope of the invention.

### In vitro studies

The inhibitory effect of compounds of Formula (I) on the sodium/glucose cotransporters SGLT1 and SGLT2 was demonstrated using the following cell-based functional assays. Stably transfected Chinese hamster ovary (CHO) cells expressing human SGLT1 or SGLT2 were prepared. Cells were treated with compounds in the presence or absence of sodium chloride and, after labeling with [¹⁴C]-α-methyl-D-glucopyranoside ([¹⁴C]-AMG) and washing, were solubilized. AMG is a non-metabolizable glucose analogue specific for sodium/glucose cotransporters. Sodium-dependent [¹⁴C]-AMG uptake was measured using scintillation counting of radioactivity [Castaneda, F., Kinne, R.K. Mol Cell Biochem 2005, 280(1-2): 91-8].

Representative compounds of Formula (I), i.e., Examples 1 to 6, presented IC₅₀ values for SGLT2 of less than 0.1 µM and IC₅₀ values for SGLT1 of greater than 10 µM.

### Platelet aggregation inhibition

Platelet aggregation inhibition was evaluated after the induction of aggregation by ADP, arachidonic acid or collagen in human platelet-rich plasma [Bertele, V. et al. Science 1983, 220(4596): 517-9; Knöfler, R. et al. Thromb Res 1995, 77(1): 69-78]. Example 1 showed inhibition of platelet aggregation at different concentrations. Platelet function was additionally tested under rheological conditions in human whole blood using a platelet function test device, PFA-100 [Frossard, M. et al. Circulation 2004, 110(11): 1392-7; Hennekens, C.H. et al. Circulation 2004, 110(12): 1706-8]. An increase (26%-30%) in aggregation time in the presence of collagen-epinephrine was noted, indicating platelet aggregation-inhibitory activity for compounds in examples 1 to 12 at 1 µM concentration under rheological conditions.

### In vivo studies in animal models

Oral administration of Example 1 produced a decrease in blood glucose concentrations in fed or fasted mice after an i.p. glucose challenge (1 g/kg) and in mice with steptozotocin-induced diabetes.

Male C57BL/6 mice weighing 25 ± 3 g were provided by Harlan. Space allocation for five animals was 29 x 18 x 13 cm. All animals were maintained in a controlled temperature (21-23 ºC) and humidity (50-70%) environment on a 12-hour light/dark cycle for 7 days in the animal facilities. Free access to standard chow for mice and reverse osmosis water were given. All aspects of this work, including housing, experimentation and disposal of animals, were performed in general accordance with the Guide for the Care and Use of Laboratory Animals.

Test substance, negative control and reference drugs (phlorizin and tolbutamide) were administered orally in fed conditions and the first glucose measurement was performed 30 minutes before the administration of 100 µL of glucose (1.8 g/kg) by the i.p. route. Then, the blood glucose concentrations were measured at 15, 30, 60, 90, and 120 minutes after the glucose injection (Glucometer and Reactive Strips Accu-chek Aviva, Roche) [Zhang, W. et al. Pharmacol Res 2011, 63: 284-93]. Free access to food and water was maintained throughout the study. Under fasting conditions, mice were deprived of food 4 hours before and during the experiment and the experimental protocol was followed as detailed above.

In mice with streptozotocin-induced diabetes [Tokunaga, H. et al. Eur J Pharmacol 1983, 87(2-3): 237-43], diabetes was induced with a single i.p. injection of streptozotocin (Sigma) 50 mg/kg body weight, freshly dissolved in NaCl 0.9% during 5 consecutive days. After 4 weeks, those mice (80%) with basal glucose concentrations under fasting (4 hours) conditions above 220 mg/dL were included in the assay. The test substance, positive and negative controls were administered by the p.o. route 30 minutes before measurement of the blood glucose concentrations at 0, 15, 30, 60, 90, and 120 minutes. Free access to water was maintained throughout the study.

Oral administration of Example 1 at 300, 30, and 3 mg/kg produced a reduction in blood glucose concentrations when compared to basal values in streptozotocin-induced diabetic mice of 18-42%, 7-26%, and 7-22%, respectively, at the experimental time points. No relevant hypoglycemic effect was observed with Example 1 at 0.1 mg/kg in streptozotocin-induced diabetic mice.

Under fed conditions, single oral doses of Example 1 of 300, 30, and 3 mg/kg showed a reduction in blood glucose concentrations when compared to basal values of 2.52% and 10.2%, 6.04% and 4.71%, and 12.01% and 9.63%, respectively, at 90 and 120 minutes after glucose challenge. Tolbutamide, employed as a positive control, exhibited a decrease of 7.76% and 3.22%, respectively, 90 and 120 minutes after glucose challenge. NaCl 0.9%, used as a negative control, showed an increase in glucose concentrations of 17.65% and 8.0%, respectively, at 90 and 120 minutes.

Oral administration of Example 1 at 3 and 0.3 mg/kg was associated with hypoglycemic activity in fasted animals. No relevant hypoglycemic effect was observed with Example 1 at 3 mg/kg in fasted mice.

### Maximum tolerated dose

The maximum tolerated dose (MTD) was assessed in mice after single oral administration of high doses of Example 1 of 1 g/kg, 500 mg/kg, and 100 mg/kg, and the development of acute toxic symptoms (mortality, convulsions, tremors, muscle relaxation, sedation, etc.) and autonomic effects (diarrhea, salivation, lacrimation, vasodilation, piloerection, etc.) was observed during the first hour. The animals were then observed again for mortality at 3, 24, 48, and 72 hours. The mice were sacrificed if they lost more than 20% of their body weight or if there were other signs of significant toxicity. The MTD obtained for Example 1 in the above experimental conditions was 1 g/kg.

### Carcinogenic assessment

Carcinogenic potential may arise from a direct genotoxic effect or nongenotoxic effects. Epigenetic factors may contribute to tumor cell proliferation by routes other than a direct interaction with DNA. In that case, xenobiotics may act as cancer promoters in species other than those employed in preclinical drug development. Cell transformation assays (CTAs), including the main in vitro CTAs --the Syrian hamster embryo (SHE) cell, BALB/c 3T3, and CH310T1/2 assays-- are experimental tools reviewed by the OECD [Environment, Health and Safety Publications, Series on Testing and Assessment nº 31. Detailed review paper on cell transformation assays for detection of chemical carcinogens. November 28, 2006]. CTA assays performed with Example 1 compared with structurally related compounds with carcinogenic potential in humans by nongenotoxic pathways showed no carcinogenic potential. No evident carcinogenic activity was observed in this assay with representative compounds of formula (I).

## Claims

1. A compound of formula (I): 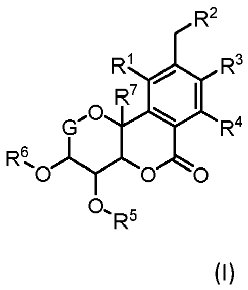 wherein
**R¹** and **R⁴** are independently selected from the group consisting of -H; halogen; -OH; C₁-C₈ alkyl optionally mono- or polysubstituted by halogen; C₁-C₈ alkyloxy optionally mono- or polysubstituted by halogen; C₃-C₁₀ cycloalkyl; -NO₂; -CN; -CO₂H; -C(O)OC₁-C₈ alkyl; -SO₂C₁-C₈ alkyl; and a 5-, 6-, or 7-membered heterocyclyl group which contains from 1 to 2 heteroatoms in the ring independently selected from the group consisting of N, O, and S;
**R²** is selected from the group consisting of C₆-C₁₀ aryl; C₃-C₁₀ cycloalkyl; a 5- or 6-membered monocyclic heteroaryl group which contains from 1 to 4 heteroatoms in the ring independently selected from the group consisting of N, O, and S; an 8-, 9-, or 10-membered bicyclic heteroaryl group which contains from 1 to 4 heteroatoms independently selected from the group consisting of N, O, and S; and a 5-, 6-, or 7-membered heterocyclyl group which contains from 1 to 2 heteroatoms in the ring independently selected from the group consisting of N, O, and S; and wherein
R² is optionally substituted by a group selected from halogen; -OH; C₁-C₈ alkyl optionally mono- or polysubstituted by halogen; C₂-C₈ alkenyl; C₂-C₈ alkynyl; C₁-C₈ alkyloxy optionally mono- or polysubstituted by halogen; C₃-C₁₀ cycloalkyl, C₆-C₁₀ aryl group which is optionally mono- or disubstituted by halogen, -CN; -NO₂; -CO₂H; -C(O)OC₁-C₈ alkyl; and -SO₂C₁-C₈ alkyl; a 5- or 6-membered monocyclic heteroaryl group which contains from 1 to 4 heteroatoms in the ring independently selected from the group consisting of N, O, and S; and an 8-, 9-, or 10-membered bicyclic heteroaryl group which contains from 1 to 4 heteroatoms in the ring independently selected from the group consisting of N, O, and S;
**R³** is selected from the group consisting of -H; halogen; C₁-C₈ alkyl optionally mono-or polysubstituted by halogen; C₂-C₈ alkenyl; C₂-C₈ alkynyl; C₁-C₈ alkyloxy optionally mono- or polysubstituted by halogen; hydroxy-C₁-C₈ alkyl; C₃-C₁₀ cycloalkyl; -OH; - NO₂; -CN; -CO₂H; -C(O)OC₁-C₈ alkyl; -SO₂C₁-C₈ alkyl; and a 5-, 6-, or 7-membered heterocyclyl group which contains from 1 to 2 heteroatoms in the ring independently selected from the group consisting of N, O, and S;
**R⁵** and **R⁶** are independently selected from the group consisting of -H; -C(O)C₁-C₈ alkyl; and -C(O)OC₁-C₈ alkyl;
**R⁷** is selected from the group consisting of-H and -OH;
**G** is selected from the group consisting of 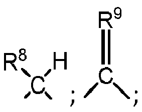 and 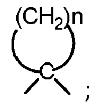 wherein
**R⁸** is selected from the group consisting of -CH₂OH; -CH₂OC₁-C₈ alkyl; -CO₂H; - C(O)OC₁-C₈ alkyl; -CH₂OC(O)-C₁-C₈ alkyl; -CH₂OC(O)O-C₁-C₈ alkyl; and C₁-C₈ alkyl optionally mono- or polysubstituted by halogen;
**R⁹** is selected from the group consisting of C₁-C₈ alkylidene; and C₃-C₁₀ cycloalkylidene;
**n** is an integer selected from 2, 3, and 4;
or a pharmaceutically acceptable salt or stereoisomer thereof.

2. A compound of formula (I) according to claim 1, wherein R¹ is selected from the group consisting of -H; -F; -Cl; -Br; -OH; -CN; -NO₂; and C₁-C₄ alkyloxy optionally mono- or polysubstituted by -F; C₁-C₄ alkyl optionally mono- or polysubstituted by - F;

3. A compound of formula (I) according to any one of claims 1 to 2, wherein R² is selected from the group consisting of C₆-C₁₀ aryl; a 5- or 6-membered monocyclic heteroaryl group which contains from 1 to 2 heteroatoms in the ring independently selected from the group consisting of N, O, and S; and an 8-, 9-, or 10-membered bicyclic heteroaryl group which contains from 1 to 4 heteroatoms independently selected from the group consisting of N, O, and S; and wherein said R² group is optionally substituted by a group selected from C₁-C₄ alkyl; C₂-C₄ alkenyl; C₂-C₄ alkynyl; C₁-C₄ alkyloxy; C₃-C₆ cycloalkyl; and C₆-C₁₀ aryl, wherein said aryl group is optionally mono- or disubstituted by halogen.

4. A compound of formula (I) according to claim 3, wherein R² is selected from the group consisting of phenyl; azulen-2-yl; thien-2-yl; and benzo[b]thien-2-yl ; and wherein said R² group is optionally substituted by a group selected from ethyl; ethynyl; ethoxy; methoxy; cyclopropyl; and 4-fluorophenyl.

5. A compound of formula (I) according to any one of claims 1 to 4, wherein R³ is selected from the group consisting of -H; halogen; C₁-C₄ alkyl; -CN; C₃-C₆ cycloalkyl; and C₁-C₄ alkyloxy, wherein said alkyl and alkyloxy groups are optionally mono- or polysubstituted by halogen.

6. A compound of formula (I) according to claim 5, wherein R³ is selected from the group consisting of -H; halogen; and C₁-C₄ alkyl.

7. A compound of formula (I) according to any one of claims 1 to 6, wherein R⁴ is selected from the group consisting of -H; C₁-C₄ alkyl; halogen; C₁-C₄ alkyloxy; and - OH.

8. A compound of formula (I) according to claim 7, wherein R⁴ is selected from the group consisting of -H; methyl; halogen; methoxy; and -OH.

9. A compound of formula (I) according to any one of claims 1 to 8, wherein R¹, R⁴, R⁵, R⁶ and R⁷ are hydrogen atoms.

10. A compound of formula (I) according to any one of claims 1 to 9, wherein G is selected from the group consisting of:
(a) 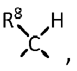 wherein R⁸ is -CH₂OH; -CH₂OC₁-C₄alkyl; or -CH₂OC(O)OC₁-C₄alkyl;
(b) 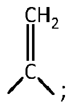 and
(c) 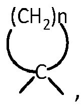 wherein n is an integer selected from 2, 3, or 4.

11. A compound of formula (I) according to claim 10, wherein G is selected from the group consisting of:
(a) 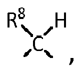 wherein R⁸ is -CH₂OH or -CH₂OC(O)OCH₂CH₃;
(b) 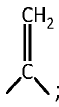 and
(c) 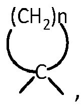 wherein n is 2.

12. A compound of formula (I) according to any one of claims 1 to 11, which is selected from group consisting of:
- (2*R*,3*S*,4*S*,4a*R*,10b*S*)-9-(4-Ethylbenzyl)-3,4-dihydroxy-2-(hydroxymethyl)-3,4,4a,10b-tetrahydropyrano[3,2-*c*][2] benzopyran-6(2*H*)-one;
- (2*R*,3*S*,4*S*,4a*R*,10b*S*)-8-Chloro-9-(4-ethoxybenzyl)-3,4-dihydroxy-2-(hydroxymethyl)-3,4,4a,10b-tetrahydropyrano[3,2-*c*][2]benzopyran-6(2H)-one;
- (2*R*,3*S*,4*S,*4a*R*,10b*S*)-8-Chloro-9-(4-ethylbenzyl)-3,4-dihydroxy-2-(hydroxymethyl)-3,4,4a,10b-tetrahydropyrano[3,2-*c*][2]benzopyran-6(2H)-one;
- (2*R*,3*S*,4*S*,4a*R*,10b*S*)-9-(4-Ethoxybenzyl)-3,4-dihydroxy-2-(hydroxymethyl)-3,4,4a,10b-tetrahydropyrano[3,2-*c*][2] benzopyran-6(2*H*)-one;
- (2*R*,3*S*,4*S*,4a*R*,10b*S*)-9-(4-Ethylbenzyl)-3,4-dihydroxy-2-(ethoxycarbonyloxymethyl)-3,4,4a,10b-tetrahydropyrano[3,2-*c*][2]benzopyran-6(2*H*)-one; and
- (2*R*,3*S*,4*S*,4a*R*,10b*S*)-3,4-Dihydroxy-2-(hydroxymethyl)-9-(4-methoxybenzyl)-3,4,4a,10b-tetrahydropyrano[3,2-*c*][2]benzopyran-6(2*H*)-one.
or a pharmaceutically acceptable salt or stereoisomer thereof.

13. A compound of Formula (I) as defined in any one of claims 1 to 12, or a pharmaceutically acceptable salt or stereoisomer thereof, for use as a medicament.

14. A compound of formula (I) as defined in any one of claims 1 to 12, or a pharmaceutically acceptable salt or stereoisomer thereof, for use in the treatment and/or prevention of a disease or condition selected from diabetes, type 2 diabetes, retinopathy, neuropathy, and nephropathy, hyperglycemia, insulin resistance, metabolic syndrome, hyperinsulinemia, hypertension, hyperuricemia, edema, dyslipidemia, chronic heart failure, atherosclerosis, obesity, and thrombosis.

15. A pharmaceutical composition comprising a compound of formula (I) as defined in any one of claims 1 to 12, or a pharmaceutically acceptable salt or stereoisomer thereof, and one or more pharmaceutically suitable excipients.

16. A combination comprising a) a compound of formula (I) as defined in any one of claims 1 to 12, or a pharmaceutically acceptable salt or stereoisomer thereof, b) another antidiabetic agent selected from the group consisting of biguanides, such as metformin and buformin; sulfonylureas, such as glibenclamide, tolbutamide, and glimepiride; meglitinides, such as nateglinide, repaglinide, and mitiglinide; thiazolidinediones, such as rosiglitazone, pioglitazone, and rivoglitazone; α-glucosidase inhibitors, such as acarbose, voglibose, and miglitol; insulin and insulin analogues, such as biphasic insulin aspart and insulin glargine; glucagon-like peptide 1 (GLP-1) and analogues, such as exenatide, liraglutide, taspoglutide, albiglutide, lixisenatide, and dulaglutide; dipeptidyl peptidase 4 (DPP IV) inhibitors, such as alogliptin, linagliptin, saxagliptin, sitagliptin, and vildagliptin; HMG-CoA reductase inhibitors, such as simvastatin, atorvastatin, lovastatin, rosuvastatin, pravastatin, and fluvastatin; GPR40 agonists, such as TAK-875; GPR119 agonists, such as PSN-821, SAR-260093, and GSK-1292263AA; GPR120 agonists; and glucokinase activators, such as piragliatin and RO-0281675; and/or c) at least a drug suitable for the treatment of hypertension, chronic heart failure or atherosclerosis, selected from the group consisting of angiotensin II receptor antagonists, such as losartan, valsartan, olmesartan, eprosartan, irbesartan, candesartan, and telmisartan; angiotensin-converting enzyme (ACE) inhibitors, such as captopril, enalapril, ramipril, lisinopril, benazepril, and perindopril; endothelin-converting enzyme (ECE) inhibitors, such as daglutril; diuretics, such as torasemide, cicletanine, chlorothiazide, chlortalidone, bendroflumethiazide, clopamide, indapamide, xipamide, hydrochlorothiazide, and amiloride; β-adrenoceptor blockers, such as propranolol, timolol, befunolol, mepindolol, nadolol, levobunolol, levobetaxolol, labetalol, sotalol, and penbutolol; calcium antagonists, such as felodipine, nisoldipine, lercanidipine, bepridil, nicardipine, nitrendipine, nimodipine, verapamil, nifedipine, barnidipine, and amlodipine; α₁-adrenoceptor antagonists, such as doxazosin, prazosin, terazosin, bunazosin, and urapidil; neutral endopeptidase inhibitors, such as ecadotril, candoxatril, and omapatrilat; and renin inhibitors, such as aliskiren.
